(19) Europäisches Patentamt
European Patent Office
Office européen des brevets

(11) Publication number: **0 448 774 B1**

(12) # EUROPEAN PATENT SPECIFICATION

(45) Date of publication of patent specification: **12.10.94**

(51) Int. Cl.$^5$: **C07D 251/70**, C08K 5/3492

(21) Application number: **90116499.6**

(22) Date of filing: **28.08.90**

(54) **Triazinic compounds.**

(30) Priority: **27.03.90 IT 1984090**

(43) Date of publication of application:
**02.10.91 Bulletin 91/40**

(45) Publication of the grant of the patent:
**12.10.94 Bulletin 94/41**

(84) Designated Contracting States:
**AT BE CH DE DK ES FR GB LI NL SE**

(56) References cited:
**EP-A- 0 281 003**
**EP-A- 0 415 371**
**US-A- 2 544 071**

(73) Proprietor: **MINISTERO DELL' UNIVERSITA' E DELLA RICERCA SCIENTIFICA E TECNOLOGICA**
**76, Lungotevere Thaon de Revel**
**I-00196 Roma (IT)**

(72) Inventor: **Cipolli, Roberto**
**V.le A. Volta 33**
**I-28100 Novara (IT)**
Inventor: **Masarati, Enrico**
**Via Pianello 321**
**I-29010 Castelnovo Valtidone (PC) (IT)**
Inventor: **Nucida, Gilberto**
**Via Mazzini 14**
**I-20098 S. Giuliano Milanese (MI) (IT)**
Inventor: **Oriani, Roberto**
**Via M. Ortigara 22**
**I-20137 Milano (IT)**

(74) Representative: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 448 774 B1

## Description

The present invention relates to triazinic compounds.

More particularly, the present invention relates to novel derivatives of 2,4,6-triamino-1,3,5-triazine which are capable of endowing thermoplastic polymers or polymers with elastomeric properties, in particular olefinic (co)polymers, with highly satisfactory self-extinguishing (flame-retardant) characteristics.

Object of the present invention are triazinic compounds of general formula (I):

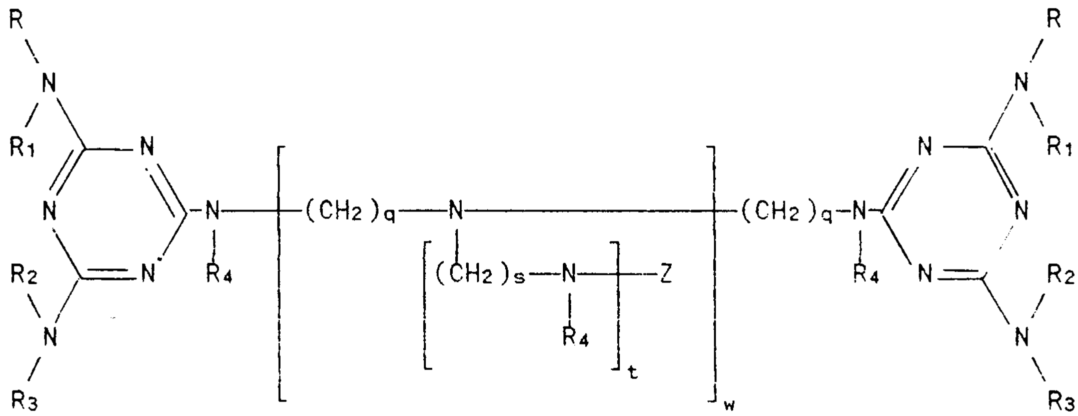

(I)

wherein:

t is 0 or 1;

<u>when t is 0:</u>

at least one of the radicals R, $R_1$, $R_2$ and $R_3$ is selected from

$\{C_nH_{2n}\}O-R_5$ and

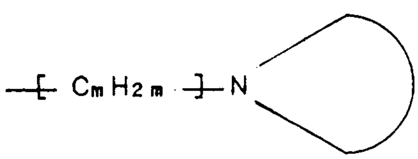

wherein:

- n = an integer of from 2 to 8, preferably from 2 to 4;
- m = an integer of from 2 to 6;
- $R_5$ = H; ($C_1$-$C_8$)-, preferably ($C_1$-$C_4$)-alkyl; ($C_2$-$C_6$)-alkenyl; $\{C_pH_{2p}\}O-R_6$, wherein <u>p</u> is an integer of from 1 to 4 and $R_6$ is H or a ($C_1$-$C_4$)-alkyl group; ($C_6$-$C_{12}$)-cycloalkyl; or ($C_6$-$C_{12}$)-alkylcycloalkyl; particularly H and ($C_1$-$C_4$)-alkyl;

and the group:

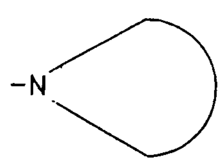

which preferably represents a saturated N-containing ring system having 2 to 8, particularly 4 to 6, carbon atoms, may represent an N-heterocyclic radical which is linked to the alkyl chain through the nitrogen atom and which is selected from aziridinyl, pyrrolidinyl, piperidinyl, morpholinyl, thiomorpholinyl, piperazinyl, 4-methyl piperazinyl and 4-ethylpiperazinyl; or in general formula (I) at least one of the moieties

$NRR_1$ and $NR_2R_3$

represents an N-heterocyclic radical which is linked to the triazine ring through the nitrogen atom and which is selected from aziridinyl, pyrrolidinyl, piperidinyl, morpholinyl, thiomorpholinyl, piperazinyl, 4-methyl piperazinyl, 4-ethyl piperazinyl, 2-methyl piperazinyl, 2,5-dimethyl piperazinyl, 2,3,5,6-tetramethyl piperazinyl, 2-ethyl piperazinyl and 2,5-diethyl piperazinyl;
the remaining radicals R to $R_3$, equal to or different from one another and having the same or different meanings in each triazine ring, are selected from the above groups and from H; ($C_1$-$C_{18}$)-alkyl; ($C_2$-$C_8$, particularly $C_2$-$C_6$)-alkenyl; ($C_6$-$C_{16}$)-cycloalkyl or ($C_6$-$C_{16}$)-alkylcycloalkyl, optionally substituted with one or more hydroxy and/or ($C_1$-$C_4$)-hydroxyalkyl groups;
<u>when t is 1:</u>
the radicals R, $R_1$, $R_2$ and $R_3$, equal to or different from one another and having the same or different meanings in each triazine ring, are selected from H; ($C_1$-$C_{18}$)alkyl; ($C_2$-$C_8$, particularly $C_2$-$C_6$)-alkenyl; ($C_6$-$C_{16}$)-cycloalkyl or($C_6$-$C_{16}$)-alkylcycloalkyl, optionally substituted with one or more hydroxy and/or ($C_1$-$C_4$)-hydroxyalkyl groups;

$-[C_nH_{2n}]-O-R_5$; and

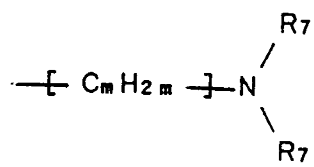

wherein:
n = an integer of from 2 to 8;
m = an integer of from 2 to 6;
$R_5$ = H; ($C_1$-$C_8$)-alkyl; ($C_2$-$C_6$)-alkenyl; $-[C_pH_{2p}]-$ 0-$R_6$, wherein <u>p</u> is an integer of from 1 to 4 and $R_6$ is H or a ($C_1$-$C_4$)-alkyl group; ($C_6$-$C_{12}$)-cycloalkyl; or ($C_6$-$C_{12}$)-alkylcycloalkyl;
the radicals $R_7$, equal to or different from one another, are selected from H; ($C_1$-$C_8$)-alkyl; ($C_2$-$C_6$)-alkenyl; ($C_6$-$C_{12}$)-cycloalkyl; ($C_6$-$C_{12}$)-alkylcycloalkyl; ($C_1$-$C_4$)-hydroxyalkyl;
and the moiety $N(R_7)_2$
may represent an N-heterocyclic radical which is linked to the alkyl chain through the nitrogen atom and which is selected from aziridinyl, pyrrolidinyl, piperidinyl, morpholinyl, thiomorpholinyl, piperazinyl, 4-methyl piperazinyl and 4-ethylpiperazinyl; or in general formula (I) at least one of the moieties

$NRR_1$ and $NR_2R_3$

represents an N-heterocyclic radical which is linked to the triazine ring through the nitrogen atom and which is selected from aziridinyl, pyrrolidinyl, piperidinyl, morpholinyl, thiomorpholinyl, piperazinyl, 4-methyl piperazinyl, 4-ethyl piperazinyl, 2-methyl piperazinyl, 2,5-dimethyl piperazinyl, 2,3,5,6-tetramethyl piperazinyl, 2-ethyl piperazinyl and 2,5-diethyl piperazinyl;
$R_4$ is hydrogen or ($C_1$-$C_4$)-alkyl;
the subscripts <u>q</u>, equal to or different from each other, are integers of from 2 to 5;
s is an integer of from 2 to 4;
w is an integer of from 1 to 5; and
Z is hydrogen or a group of general formula

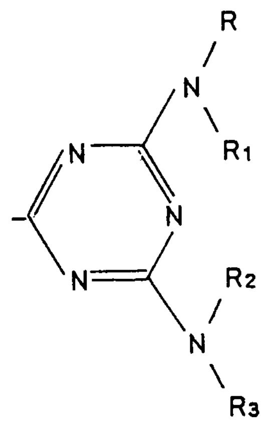

wherein R to $R_3$ are defined as above and for t = 0 or t = 1, respectively, and the specific meanings of Z can be different in each repeating unit. Compounds having an asymmetrical structure in that the radicals R to $R_3$ have different meanings in each triazine ring are also meant to be included in the above definition.

Specific examples of radicals R, $R_1$,$R_2$ and $R_3$ in the above general formula (I) are:
methyl; ethyl; propyl; isopropyl; n-butyl; isobutyl; tert - butyl; n-pentyl; isopentyl; n-hexyl; tert -hexyl; octyl; tert -octyl; decyl; dodecyl; octadecyl; ethenyl; propenyl; butenyl; isobutenyl; hexenyl; octenyl; cyclohexyl; propylcyclohexyl; butyl cyclohexyl; decyl cyclohexyl; hydroxycyclohexyl;hydroxyethyl cyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethyl hexyl; 7-hydroxyheptyl; 7-hydroxyoctyl; 2-methoxy ethyl; 2-methoxy propyl; 3-methoxy propyl; 4-methoxy butyl; 6-methoxy hexyl; 7-methoxy-heptyl; 7-methoxy octyl; 2-ethoxy ethyl; 3-ethoxy propyl; 4-ethoxy butyl; 3-propoxy propyl; 3-butoxy propyl; 4-butoxy-butyl; 4-isobutoxy butyl; 5-propoxy pentyl; 2-cyclohexyloxy-ethyl; 2-ethenyloxy ethyl; 2-(N,N-dimethylamino) ethyl; 3-(N,N-dimethylamino) propyl; 4-(N,N-dimetnylamino) butyl; 5-(N,N-dimethylamino) pentyl; 4-(N,N-diethylamino) butyl; 5-(N,N-diethylamino) pentyl; 5-(N,N-di-isopropylamino) pentyl; 3-(N-ethylamino) propyl; 4-(N-methylamino) butyl; 4-(N,N-di-propylamino) butyl; 2-(N,N-diisopropylamino) ethyl; 6-(N-hexenylamino) hexyl; 2-(N-ethenylamino) ethyl; 2-(N-cyclohexylamino) ethyl; 2-(N-2-hydroxyethylamino) ethyl; 2-(2-hydroxyethoxy) ethyl; 2-(2-methoxyethoxy) ethyl; etc. Examples of polyvalent radicals of formula

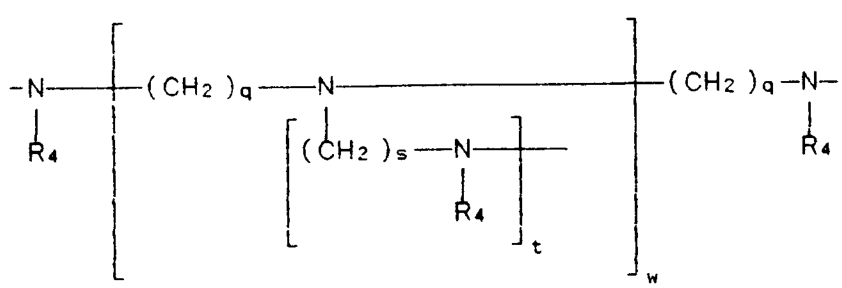

are those which derive, by elimination of a hydrogen atom from each reacted amino group, from the following polyamine compounds:
bis (2-aminoethyl) amine; bis (3-aminopropyl) amine: bis (4-aminobutyl) amine: bis (5-aminopentyl) amine; bis [2-(N-methylamino)ethyl] amine; 2-N-butyl bis (2-aminoethyl)-amine; bis [3-(N-methylamino) propyl]-amine; N-(3-aminopropyl)-1,5-diamino pentane; N-(4-aminobutyl)-1,5-diamino pentane; tris (2-aminoethyl) amine; tris (3-aminopropyl) amine; tris (4-aminobutyl) amine; tris [2-(N-ethylamino) ethyl] amine; N,N'-bis (2-aminoethyl)-1,2-diamino ethane; N,N'-bis (3-aminopropyl)-1,3-diamino-propane; N,N'-bis (2-aminoethyl)-1,3-diamino propane; N,N'-bis (3-aminopropyl)-1,2-diamino ethane; N,N'-bis (3-aminopropyl-1,4-diaminobutane; bis [2-(2-aminoethyl)aminoethyl] amine; N,N' bis-[2-(2-aminoethyl) aminoethyl]-1,2-diamino ethane; N,N'-bis [3-(2-aminoethyl) aminopropyl]-1,2-diamino ethane; N,N,N',N'-tetrakis (2-aminoethyl)-1,2-diamino ethane; etc.

Specific compounds comprised within the scope of general formula (I) are reported in the examples below.

The compounds of general formula (I) may be prepared by reacting, preferably at temperatures of from about 0 to about 10 °C and at a pH value of from 5 to 7, a halide of cyanuric acid, such as, e.g., cyanuric chloride, in a suitable solvent (such as, e.g., acetone, water and methylene chloride) with an amine of general formula (II):

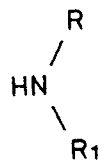

(II)

wherein R and $R_1$ have the meanings given above, in the presence or absence (depending on the molar ratio used in the reaction) of an acid acceptor (such as, e.g., NaOH, $NaHCO_3$, $Na_2CO_3$ and triethylamine), resulting in the formation of the intermediate of the general formula (III):

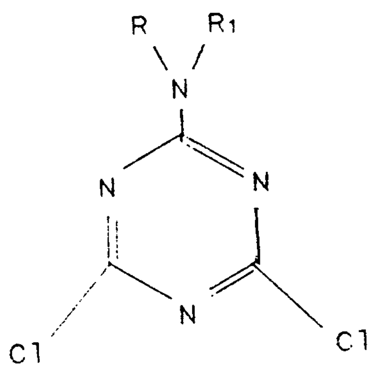

(III)

Said intermediate, isolated or not isolated from the reaction mixture, may subsequently be reacted, under conditions similar or identical to those specified hereinabove but preferably at a temperature of from about 10 to about 50 °C, with an amine of general formula (IV):

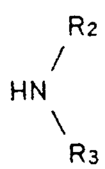

(IV)

wherein $R_2$ and $R_3$ have the meanings given above, resulting in the formation of the intermediate of general formula (V):

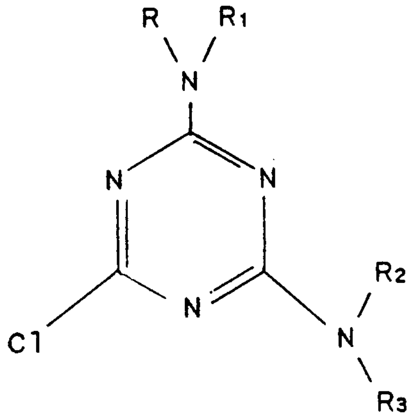

(V)

The intermediate (V), isolated or not isolated and in a number of mols smaller than or equal to (2 + w), may in turn be reacted, under the same conditions as in the case of the two first reaction steps, but preferably operating at a higher temperature than that of the preceding step, e.g., of from about 70 to about 150 °C (and hence using a solvent which is compatible with such temperatures, such as, e.g., water, toluene, xylene and dimethylformamide) with one mol of a polyamine of general formula (VI):

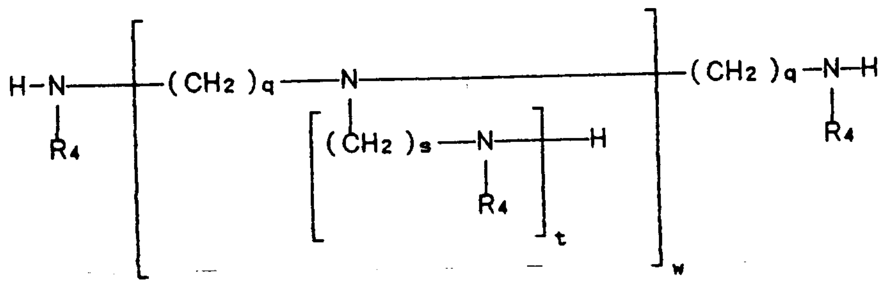

(VI)

wherein $R_4$, q, s, t and w have the meanings given hereinabove, affording the compounds of general formula (I) as the end products.

In case compounds of general formula (I) wherein the groups $NRR_1$ and $NR_2R_3$ are the same are desired, the above process may be carried out by reacting cyanuric chloride with two mols of an amine of general formula (II) under the same conditions as disclosed hereinabove in order to obtain the intermediate of general formula (V).

An alternative method comprises the reaction of a number of moles lower than or equal to (2 + w) of a halide (such as, e.g., the chloride) of cyanuric acid with one mol of a polyamine of general formula (VI) as defined above, under the same conditions as disclosed hereinabove, preferably at a temperature of from about 0 to about 10 °C, so as to yield the intermediate of general formula (VII):

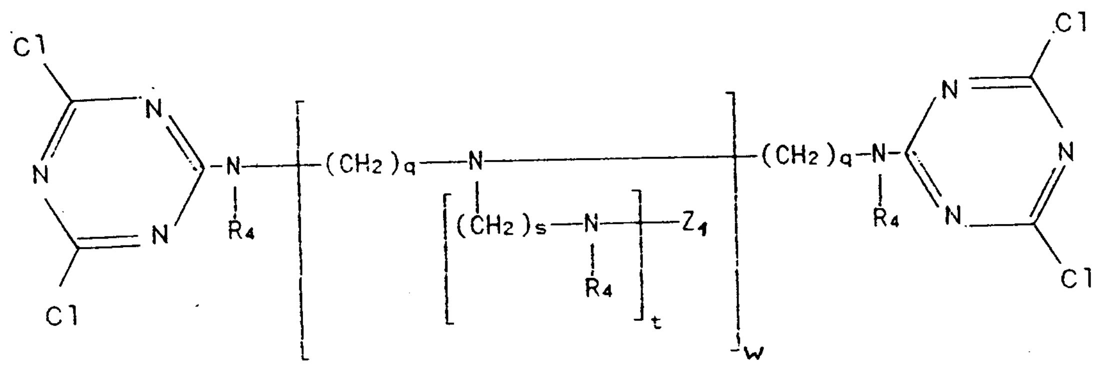

(VII)

wherein $Z_1$ is hydrogen or a group of formula

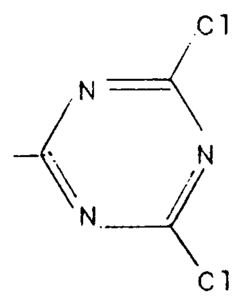

and its meaning can be different in each repeating unit.

Said intermediate, isolated or not isolated, may be reacted:

(a) with a number of mols lower than or equal to (2 + w) of an amine of general formula (II), preferably at a temperature of from about 40 to about 80 °C, to yield the intermediate of general formula (VIII):

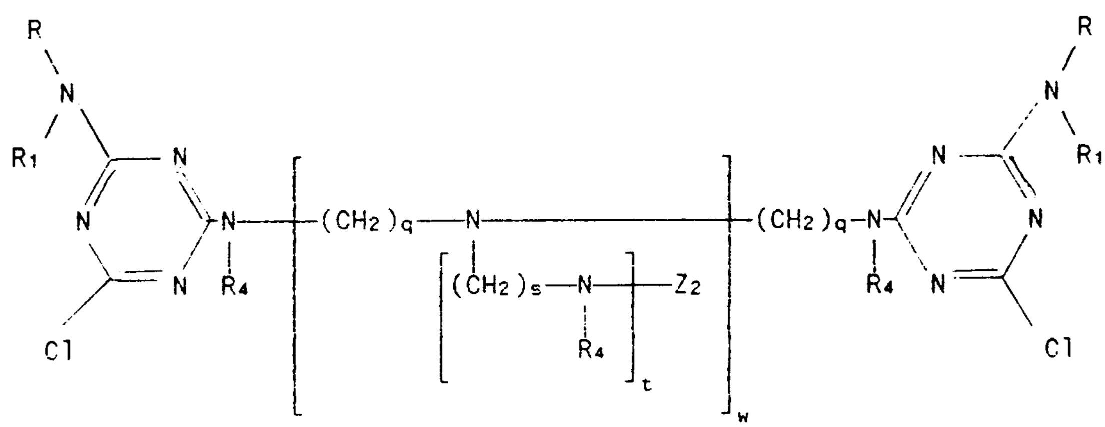

(VIII)

wherein $Z_2$ is hydrogen or a group of formula

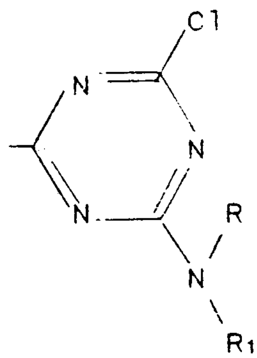

and its meaning can be different in each repeating unit; which intermediate, isolated or not isolated, may be reacted with a number of mols lower than or equal to (2+w) of an amine of general formula (IV), preferably at a temperature of from about 80 to about 150°C and under conditions similar to those disclosed hereinabove; or

(b) with a number of mols lower than or equal to 2(2+w) of an amine of general formula (II), preferably at a temperature of from about 80 to about 150°C, under conditions similar to those disclosed hereinabove.

High quality products of general formula (I) are generally obtained as white crystalline powders, which can be used for the preparation of self-extinguishing polymeric compositions without further purification.

The following non-limitative examples are to further illustrate the present invention.

Example 1

184.5 g of cyanuric chloride and 1300 ml of methylene chloride were charged to a 3 l-reactor equipped with stirrer, thermometer, charging funnel, reflux condenser and cooling bath.

With the reaction mixture being externally cooled, 87.2 g of morpholine and 40 g of sodium hydroxide, dissolved in 150 g of water, were simultaneously added to the reaction mixture over 3 hours, the pH value of said reaction mixture being kept within the range of from 5 to 7, and the temperature being kept within the range of from 0 to 3°C.

The reaction mass was kept at 0 to 3°C for a further 3 hours, and then the aqueous phase was separated.

Following distillation of methylene chloride, 230 g of intermediate (IX):

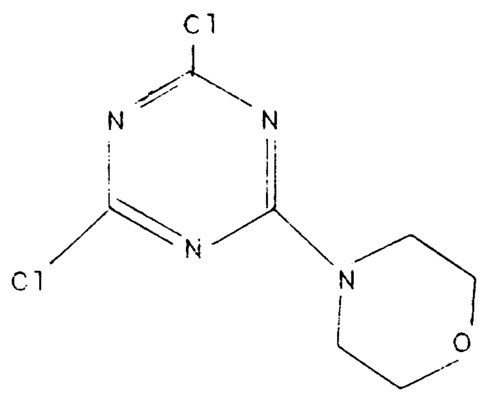

(IX)

were obtained as white crystalline powder having a melting point (m.p.) of 155-157°C and a chlorine content of 29.87% (theoretical value: 30.21%).

A 1 l-reactor, equipped as the preceding one but additionally provided with a heating bath, was charged with 200 g of an aqueous solution at 30% by weight of ammonia, 200 ml of water and 141 g of intermediate (IX).

The reaction mixture was heated to 50°C and was kept at this temperature for 7 hours. It then was cooled to room temperature and the resulting precipitated solid product was filtered off and washed with water.

After drying the filter cake, 116 g of intermediate (X):

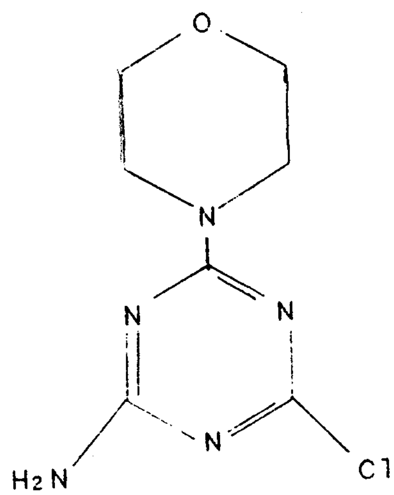

(X)

were obtained as white crystalline powder having a m.p. of 189-191 °C and a chlorine content of 16.28% (theoretical value: 16.47%).

The structure of compounds (IX) and (X) was confirmed by I.R. spectroscopic analyses.

400 ml of xylene, 64.7 g of intermediate (X) and 10.3 g of diethylene triamine were charged to a 1 l-reactor (equipped as disclosed above).

The reaction mixture was heated to 100 °C and kept at this temperature for 2 hours. Then 12 g of sodium hydroxide were added and the reaction mixture was heated to boiling.

The reaction mass was refluxed for 24 hours and then was cooled to room temperature. The precipitated solid product was filtered off and the filter cake was thoroughly washed with water.

By oven-drying at 100 °C, 56.7 g of product of formula:

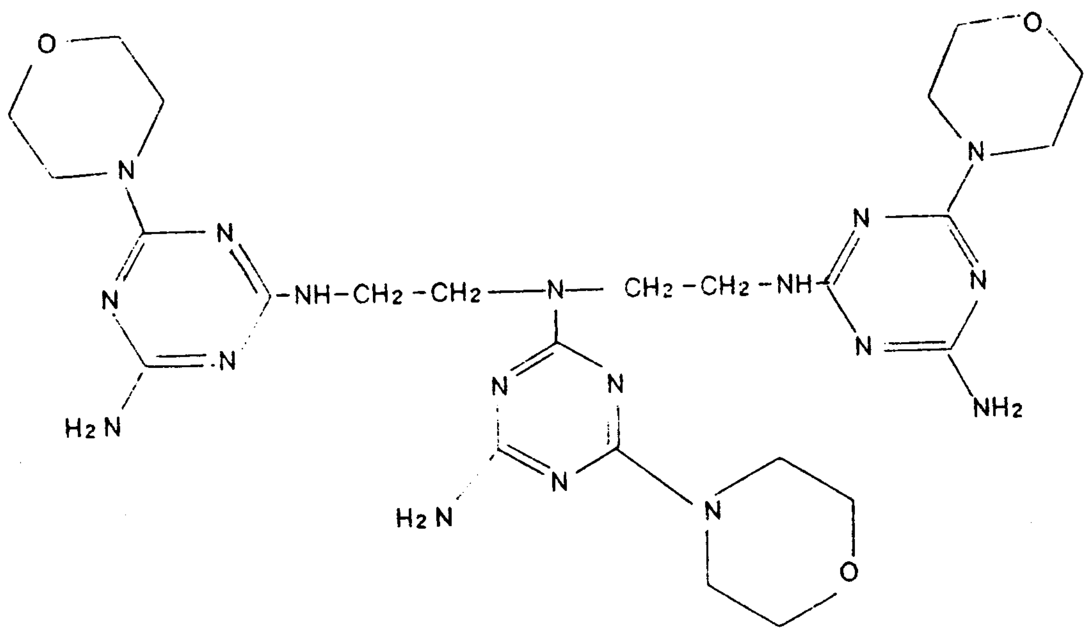

were obtained as crystalline powder having a m.p. of 207-208 °C.

Example 2

184.5 g of cyanuric chloride and 800 ml of acetone were charged to a 3 l-reactor equipped with stirrer, thermometer, charging funnel, reflux condenser and heating bath.

The reaction mixture was stirred and heated to 40 °C until a solution was obtained. Then, with the temperature being kept constant at 40 °C, 284 g of an ammonia solution at 30% by weight was added over 1.5 hours.

The reaction mixture was subsequently heated to 45 °C and kept at this temperature for 4 hours.

After cooling, the precipitated solid was filtered off and the filter cake was washed with water on the same filter.

After oven-drying at 100 °C, 113 g of intermediate (XI):

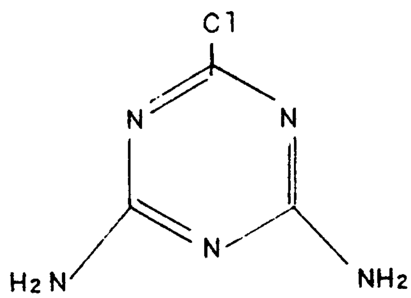

(XI)

were obtained as white, infusible, crystalline powder having a chlorine content of 24.2% (theoretical value: 24.4%).

The structure of this compound was also confirmed by I.R. spectroscopic analysis.

500 ml of water, 87.3 g of intermediate (XI) and 29.2 g of tris (2-aminoethyl) amine were charged to a 1 l-reactor equipped as disclosed hereinabove.

The reaction mixture was heated to 50 °C and kept at this temperature for 1 hour.

Then 24.0 g of sodium hydroxide, dissolved in 50 ml of water, were added over three hours and the reaction mixture was simultaneously heated to boiling.

The reaction mass was refluxed for about 10 hours and then cooled to room temperature and the precipitated solid product was filtered off.

The filter cake was washed with water and dried.

89.4 g of product of formula:

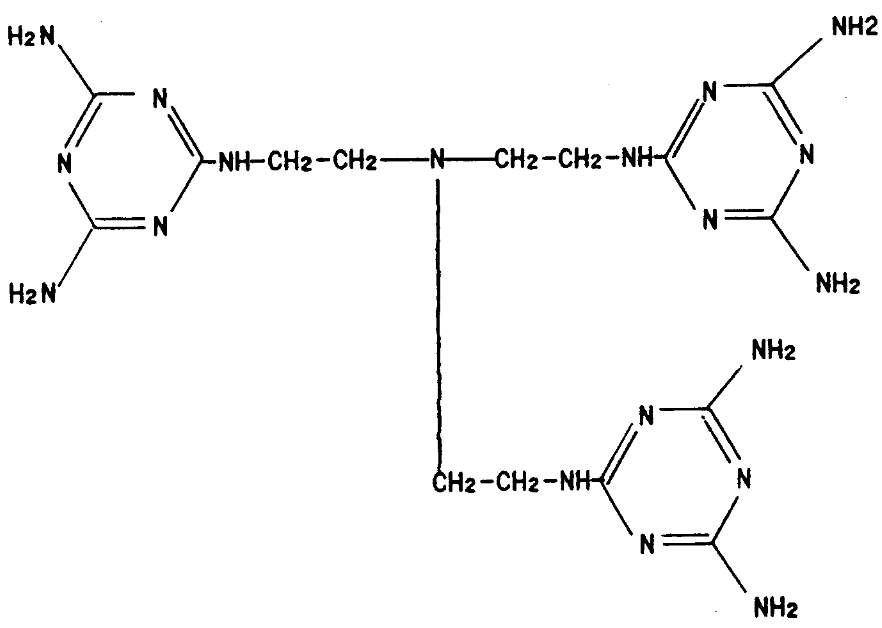

were obtained as white crystalline powder having a m.p. of 125-130 °C.

Example 3

184.5 g of cyanuric chloride and 1300 ml of methylene chloride were charged to a 3 l-reactor equipped in the same way as that of example 1.

With the reaction mixture being externally cooled, 75 g of 2-methoxyethylamine and 40 g of sodium hydroxide, dissolved in 150 ml of water, were simultaneously added to the reaction mixture over 3 hours, with the pH value of said reaction mixture being kept within the range of from 5 to 7, and the temperature thereof being kept within the range of from 0 to 3 °C.

The reaction mixture was kept at a temperature of 0 to 3 °C for a further 3 hours, and the aqueous phase was then separated.

The organic solution was treated with two 200 ml-portions of water, the aqueous phase being separated each time.

Upon distilling off methylene chloride, 217.5 g of intermediate (XII):

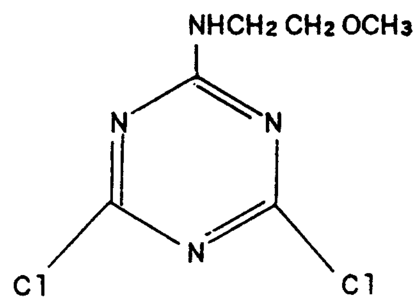

(XII)

were obtained as white crystalline powder having a m.p. of 73-75 °C and a chlorine content of 31.68% (theoretical value: 31.84%).

A 1 l-reactor equipped with stirrer, thermometer, charging funnel, reflux condenser and heating bath, was charged with 400 ml of acetone and 133.8 g of intermediate (XII).

The reaction mixture was heated with stirring to 40 °C until a solution was obtained. Then, with the temperature of the reaction mixture being still kept at 40 °C, 102 g of an aqueous solution of ammonia at 30% by weight were added over 30 minutes.

The resulting reaction mixture was subsequently heated to 45 °C and kept at this temperature for 4 hours.

After cooling to 10 °C, the precipitated solid product was filtered off and the filter cake was washed with cold water on the same filter.

After oven-drying at 100 °C, 114 g of the intermediate product (XIII):

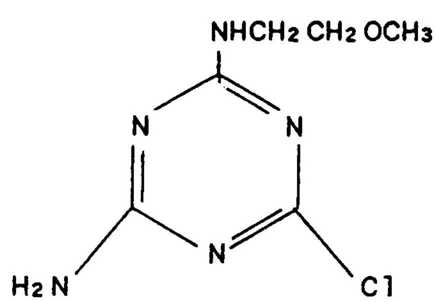

(XIII)

were obtained as crystalline powder having a m.p. of 195-197 °C and a chlorine content of 17.18% (theoretical value: 17.44%).

The structure of the intermediates (XII) and (XIII) also was confirmed by I.R.-spectroscopic analyses.

500 ml of ortho-dichlorobenzene, 91.6 g of intermediate (XIII) and 21.9 g of tris(2-aminoethyl) amine were placed in a 1 l-reactor equipped in the same way as that of the preceding example.

The reaction mixture was heated to 100 °C and was kept at that temperature for 2 hours. Then, 18 g of sodium hydroxide were added and the temperature was raised to 140 °C. The reaction mass was kept at 140 °C for 16 hours and then cooled to room temperature, whereafter the precipitated solid product was filtered off. The filter cake was thoroughly washed with water.

After drying the filter cake, 88.2 g of product of formula:

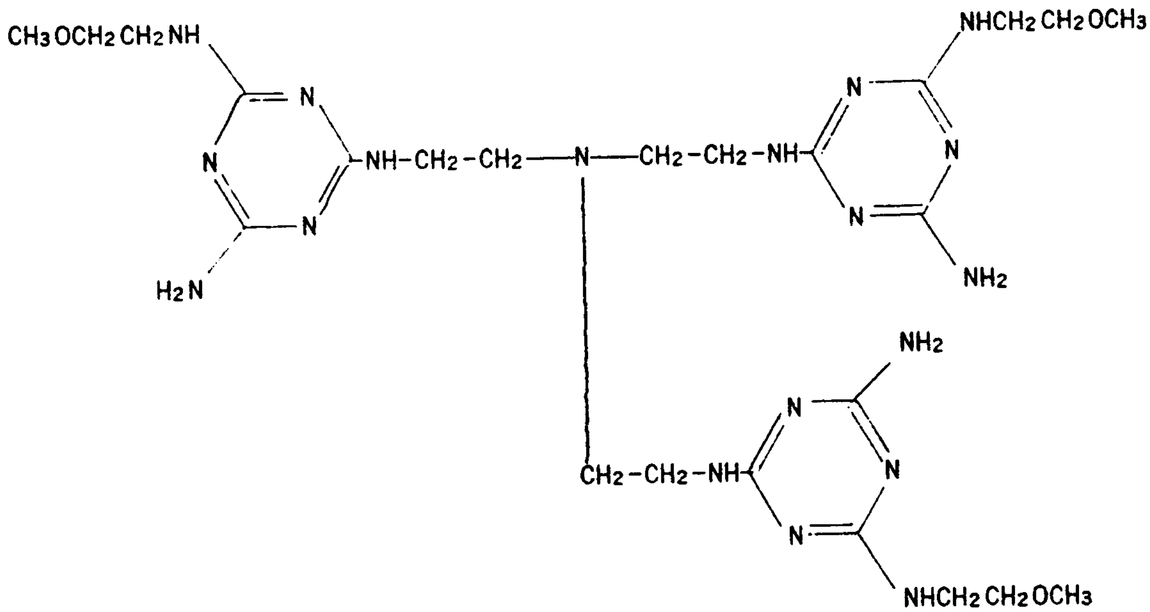

were obtained as crystalline powder having a m.p. of 190-195 °C.

Example 4

500 ml of xylene, 86.2 g of intermediate (X) and 15.1 g of tetraethylenepentamine were charged to the 1 l-reactor described in example 3.

The reaction mass was heated to 80 °C and was kept at that temperature for 2 hours. 16 g of sodium hydroxide were then added and the temperature was raised to 110 °C.

The reaction mass was kept at 110 °C for 18 hours and then was cooled to room temperature. The precipitated solid product was filtered off and the filter cake was thoroughly washed with water.

After oven-drying the cake at 100 °C, 82.6 g of product of formula:

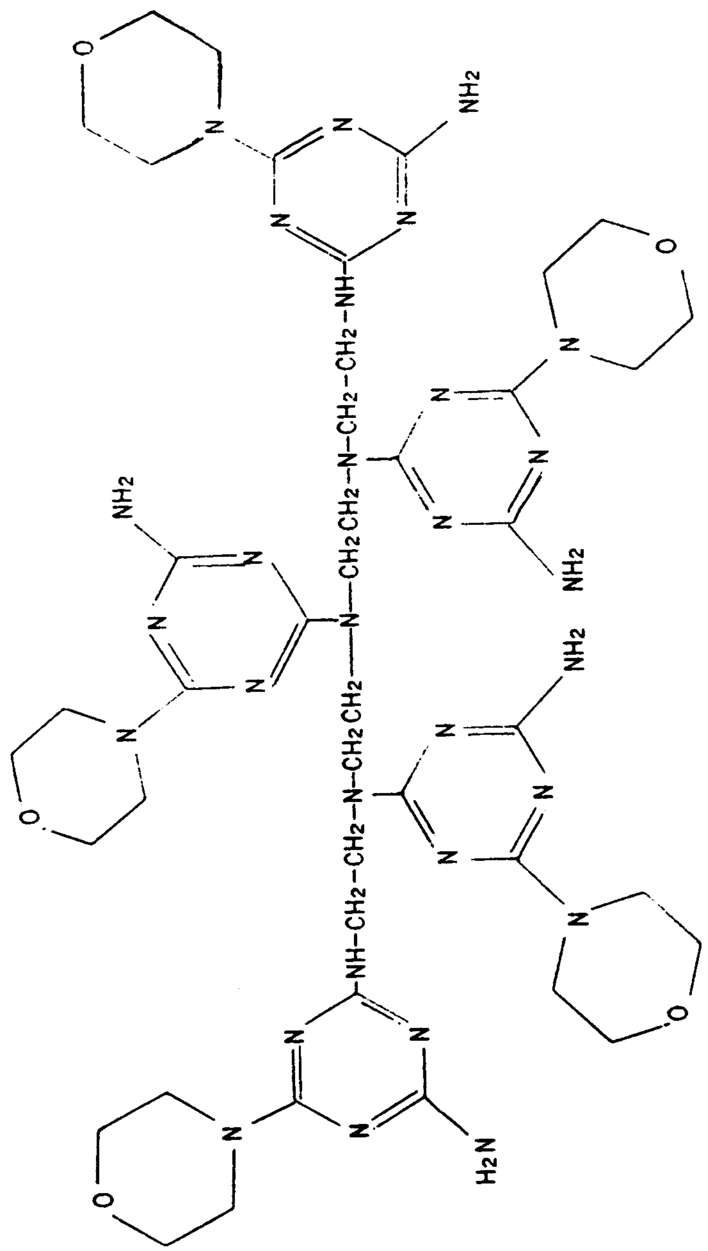

were obtained as crystalline powder having a m.p. of 178-183 °C.

Example 5

55.3 g of cyanuric chloride and 300 ml of acetone were charged to the 1 l-reactor described in example 1.

With the reaction mixture being kept at 0-5 °C by means of external cooling, 10.3 g of diethylenetriamine, dissolved in 200 ml of acetone, were added over 1 hour.

With the reaction temperature being still kept at 0 to 5 °C, 12 g of sodium hydroxide, dissolved in 100 g of water, were added.

The reaction mixture was stirred, at 5 °C, for a further 4 hours and then 200 g of cold water were added. The precipitated solid product was filtered off and the filter cake was washed with water on the same filter.

After drying, 45.6 g of the intermediate (XIV):

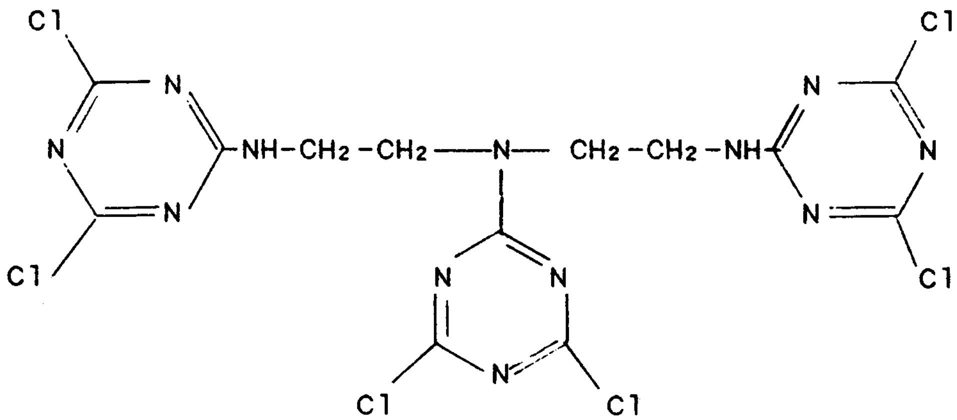

(XIV)

were obtained as crystalline white powder having a m.p. higher than 300 °C and a chlorine content of 38.46% (theoretical value: 38.94%).

To the same 1 l-reactor, this time equipped with a heating bath, 500 ml of xylene and 32.8 g of intermediate (XIV) were charged.

The reaction mixture was heated to 80 °C and subsequently 31.3 g of morpholine and then 14.4 g of sodium hydroxide in 50 g of water were added within 4 hours.

The temperature was gradually increased, with water being removed by azeotropic distillation, until the solvent boiling temperature was reached.

The reaction mixture was kept under reflux conditions for 8 hours and then was cooled to room temperature and filtered. The filter cake was thoroughly washed with water.

After drying, 43.1 g of product of formula:

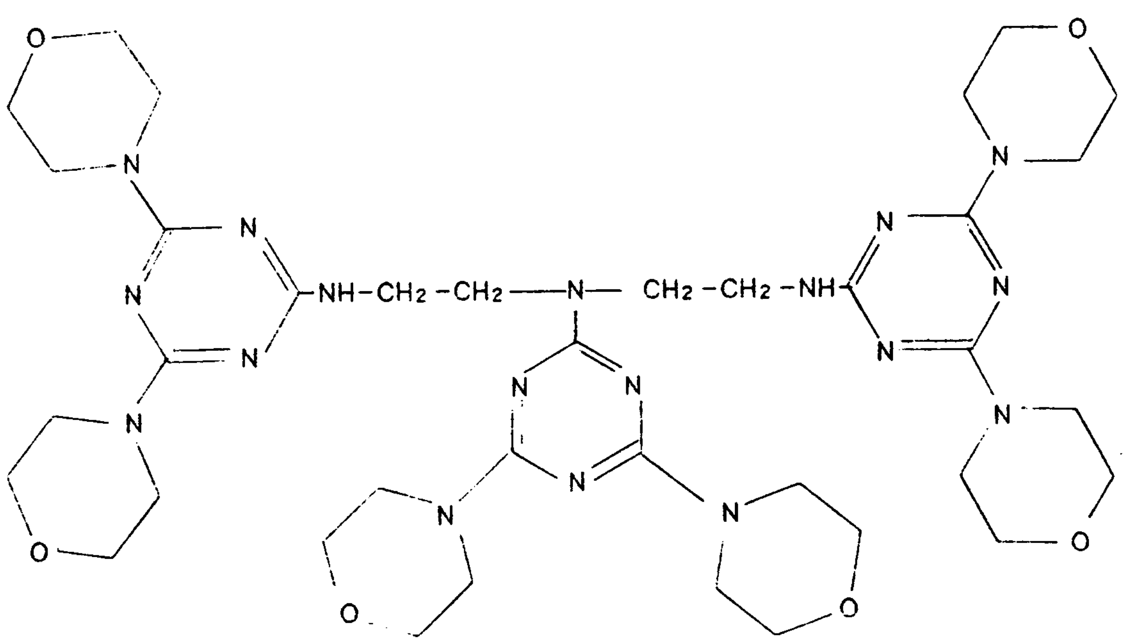

were obtained as white crystalline powder having a m.p. of 277-280 °C.

Example 6

450 ml of water, 91.6 g of intermediate (XIII) and, with stirring, 15.4 g of diethylenetriamine were charged to the 1 l-reactor of example 2.

The reaction mass was heated to 80 ° C and was kept at that temperature for 3 hours.

18 g of sodium hydroxide, dissolved in 30 ml of water, were then added and the temperature of the reaction mixture was raised to its boiling point. The reaction mass was refluxed for 16 hours and then cooled to about 10 ° C. The precipitated product was filtered off and the filter cake was washed with cold water on the filter.

Upon oven-drying the cake at 100 ° C, 77.9 g of product of formula:

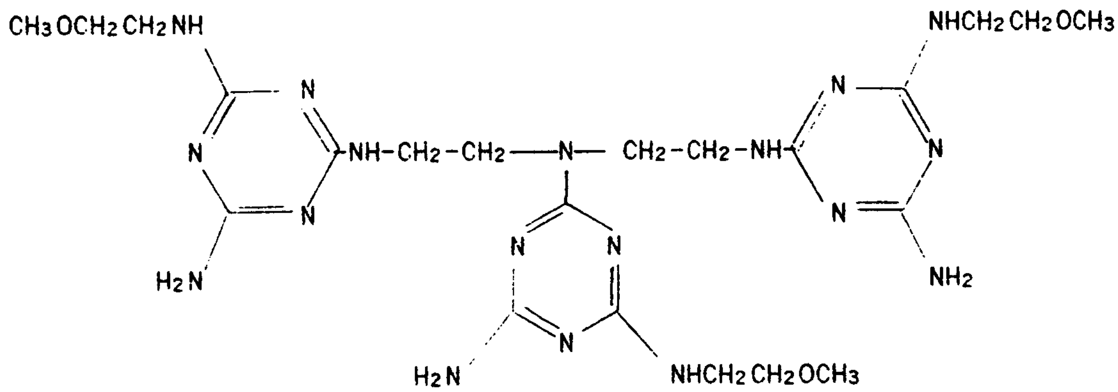

were obtained as crystalline powder having a m.p. of 296-299 ° C.

Example 7

400 ml of water, 86.2 g of intermediate (X) and, with stirring, 20.6 g of diethylenetriamine were charged to the 1 l-reactor of example 6.

The reaction mass was heated to 80 ° C and kept at that temperature for 2 hours, whereafter 16 g of sodium hydroxide, dissolved in 30 ml of water, were added.

The temperature of the reaction mixture was raised to the boiling point and the reaction mass was refluxed for 14 hours.

Then, by operating in the same way as in example 6, 86.2 g of product of formula:

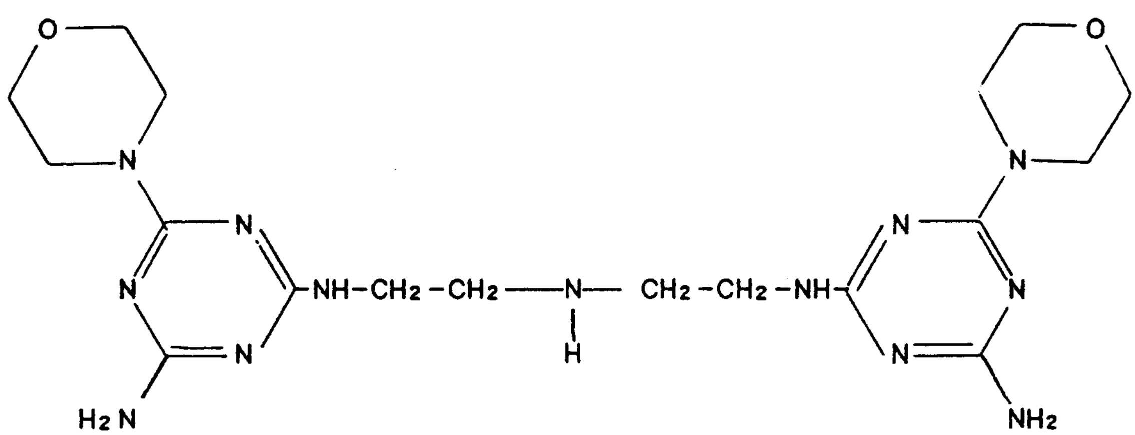

were obtained as crystalline powder having a m.p. of 198-201 ° C.

Examples 8 - 16

By operating under conditions analogous to those disclosed in examples 1 to 7, the compounds of general formula (I) reported in Table 1 were prepared,wherein Z is a group of general formula

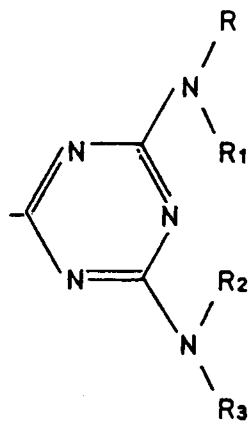

Table 1

| Example No. | R⟵N⟶R₁ | | R₂⟵N⟶R₃ | | 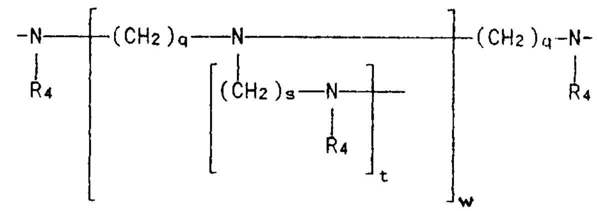 | Melting point (°C) |
|---|---|---|---|---|---|---|
| 8 | $CH_2CH_2OCH_3$ | H | 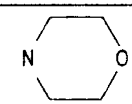 | | $-NH-(CH_2)_3-N-(CH_2)_3-NH-$ | 242-246 |
| 9 | 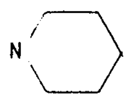 | | H | H | $-NH-CH_2CH_2-N-CH_2CH_2-NH-$ | 259-262 |
| 10 | 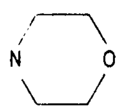 | | $n-C_4H_9$ | H | $-NH-CH_2CH_2-N-CH_2CH_2-NH-$ | 135-142 |
| 11 | $CH_2CH_2CH_2$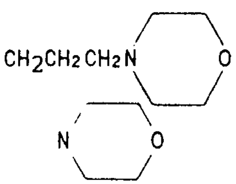 | H | H | H | $N-(CH_2CH_2NH-)_3$ | 172-177 |
| 12 | | | $CH_2-CH=CH_2$ | H | $-NH-(CH_2)_4-N-(CH_2)_3-NH-$ | 157-161 |

(cont.d) Table 1

| Example No. | R(N)R₁ | | R₂(N)R₃ | | 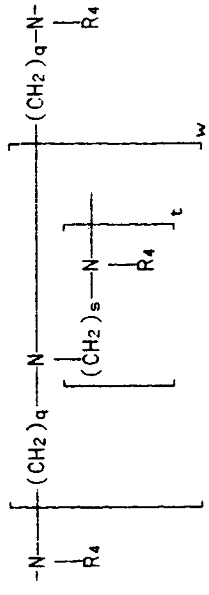 | Melting point (°C) |
|---|---|---|---|---|---|---|
| 13 | $CH_2CH_2OCH=CH_2$ | H | H | H | $-NH-(CH_2)_3-N-(CH_2)_2-N-(CH_2)_3-NH-$ | 199-204 |
| 14 | $(CH_2)_5OH$ | H | H | H | $-NH-CH_2CH_2-N-CH_2CH_2-NH-$ | 274-277 |
| 15 | 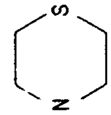 | | t-$C_8H_{17}$ | H | $-NH-CH_2CH_2-N-CH_2CH_2-NH-$ | 122-127 |
| 16 | $CH_2CH_2OCH_3$ | $CH_2CH_2OCH_3$ | H | H | $-NH-CH_2CH_2-N-CH_2CH_2-NH-$ | 213-217 |

Example 17

75.0 g of isotactic polypropylene flakes having a melt flow index of 12 and a content of 96% by weight of a fraction insoluble in boiling n-heptane; 8.0 g of the product of Example 1; 16.0 g of ammonium

polyphosphate (Exolit®422, manufactured by Hoechst AG); 0.67 g of dilaurylthiopropionate and 0.33 g of pentaerythritol tetra [3-(3,5-di-tert -butyl-4-hydroxyphenyl) propionate] were mixed with one another and the resulting mixture was moulded on a MOORE platen press, with a moulding time of 7 minutes and a moulding pressure of 40 kg/cm$^2$.

Specimen were obtained in the form of small slabs having a thickness of about 3 mm. On said specimen the level of self-extinguishment was determined by measuring the oxygen index (L.O.I. according to ASTM D-2863/77) on a STANTON REDCROFT apparatus, and by applying the "vertical burning test" which makes it possible to classify the material according to three ratings (V-0, V-1 and V-2, compare UL 94 standards published by Underwriters Laboratories, U.S.A.).

The following results were obtained:
L.O.I. = 36.4
UL 94 : Class V-0.

**Claims**

**Claims for the following Contracting States : AT, BE, CH, DE, DK, FR, GB, LI, NL, SE**

**1.** Triazinic compounds of general formula (I):

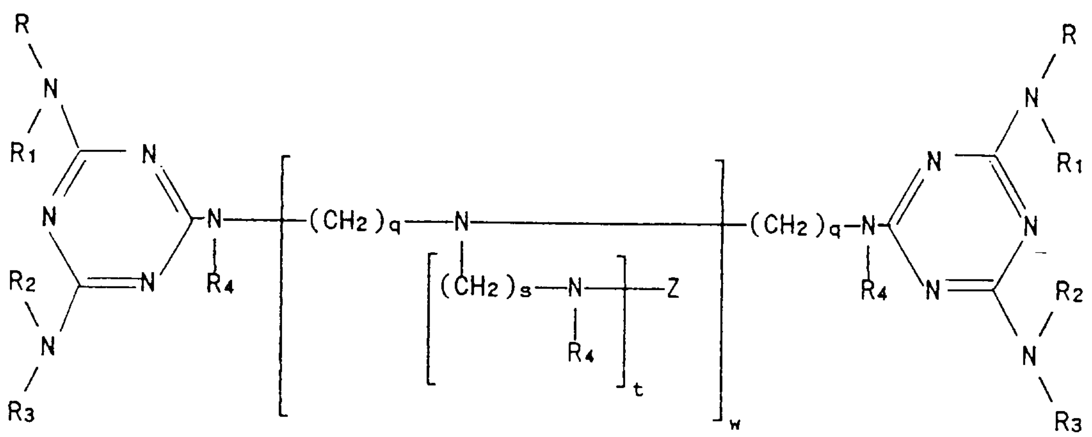

(I)

wherein:
t is 0 or 1;
<u>when t is 0:</u>
at least one of the radicals R, $R_1$, $R_2$ and $R_3$ is selected from

$-[C_nH_{2n}]-O-R_5$; and

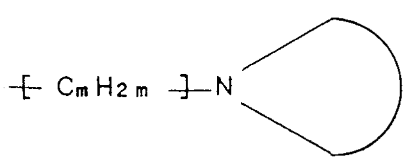

wherein:

n = an integer of from 2 to 8;
m = an integer of from 2 to 6;
$R_5$ = H; $(C_1-C_8)$-alkyl; $(C_2-C_6)$-alkenyl; $-[C_pH_{2p}]-O-R_6$, wherein <u>p</u> is an integer of from 1 to 4 and $R_6$ is H or a $(C_1-C_4)$-alkyl group; $(C_6-C_{12})$-cycloalkyl; or $(C_6-C_{12})$-alkylcycloalkyl;

and the group:

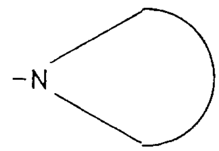

represents an N-heterocyclic radical which is selected from aziridinyl, pyrrolidinyl, piperidinyl, morpholinyl, thiomorpholinyl, piperazinyl, 4-methyl piperazinyl and 4-ethylpiperazinyl, and which is linked to the alkyl chain through the nitrogen atom;
or in general formula (I) at least one of the moieties

$NRR_1$ and $NR_2R_3$

represents an N-heterocyclic radical which is selected from aziridinyl, pyrrolidinyl, piperidinyl, morpholinyl, thiomorpholinyl, piperazinyl, 4-methyl piperazinyl, 4-ethyl piperazinyl, 2-methyl piperazinyl, 2,5-dimethyl piperazinyl, 2,3,5,6-tetramethyl piperazinyl, 2-ethyl piperazinyl and 2,5-diethyl piperazinyl, and which is linked to the triazine ring through the nitrogen atom;
the remaining radicals R to $R_3$, equal to or different from one another and having the same or different meanings in each triazine ring, are selected from the above groups and from H; ($C_1$-$C_{18}$)-alkyl; ($C_2$-$C_8$)-alkenyl; ($C_6$-$C_{16}$)-cycloalkyl or ($C_6$-$C_{16}$)-alkylcycloalkyl, optionally substituted with one or more hydroxy and/or ($C_1$-$C_4$)-hydroxyalkyl groups;
<u>when t is 1:</u>
the radicals R to $R_3$, equal to or different from one another and having the same or different meanings in each triazine ring, are selected from H; ($C_1$-$C_{18}$)-alkyl; ($C_2$-$C_8$)-alkenyl; ($C_6$-$C_{16}$)-cycloalkyl or ($C_8$-$C_{16}$)-alkylcycloalkyl, optionally substituted with one or more hydroxy and/or ($C_1$-$C_4$)-hydroxyalkyl groups;

$\{C_nH_{2n}\}O\text{-}R_5$; and

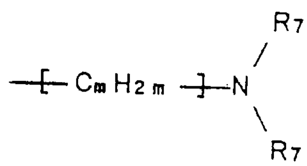

wherein:
n, m and $R_5$ are defined as above;
the radicals $R_7$, equal to or different from one another are selected from H; ($C_1$-$C_8$)-alkyl; ($C_2$-$C_6$)-alkenyl; ($C_6$-$C_{12}$)-cycloalkyl; ($C_6$-$C_{12}$)-alkylcycloalkyl; and ($C_1$-$C_4$)-hydroxyalkyl; and the moiety $N(R_7)_2$ may represent an N-heterocyclic radical which is selected from aziridinyl, pyrrolidinyl, piperidinyl, morpholinyl, thiomorpholinyl, piperazinyl, 4-methyl piperazinyl and 4-ethyl piperazinyl, and which is linked to the alkyl chain through the nitrogen atom;
or in general formula (I) at least one of the moieties

$NRR_1$ and $NR_2R_3$

represents an N-heterocyclic radical which is selected from aziridinyl, pyrrolidinyl, piperidinyl, morpholinyl, thiomorpholinyl, piperazinyl, 4-methyl piperazinyl, 4-ethyl piperazinyl, 2-methyl piperazinyl, 2,5-dimethyl piperazinyl, 2,3,5,6-tetramethyl piperazinyl, 2-ethyl piperazinyl and 2,5-diethyl piperazinyl, and which is linked to the triazine ring through the nitrogen atom;
$R_4$ is hydrogen or ($C_1$-$C_4$)-alkyl;
the subscripts <u>q</u>, equal to or different from each other, are integers of from 2 to 5;
s is an integer of from 2 to 4;
w is an integer of from 1 to 5; and
Z represents hydrogen or a group of general formula:

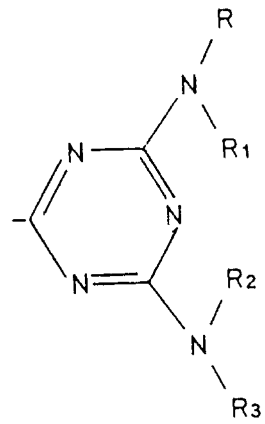

wherein R to $R_3$ are as defined above for t = 0 or t = 1, respectively, and the specific meanings of Z can be different in each repeating unit.

**2.** Process for preparing the compounds of general formula (I) according to claim 1, wherein a polyamine of general formula (VI):

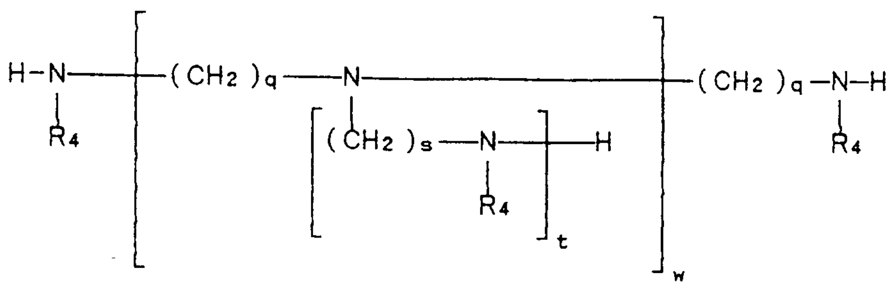

(VI)

wherein $R_4$, q, s, t and w have the meanings given in claim 1, is reacted with at the most (2 + w) moles per mole of polyamine of an intermediate of general formula (V):

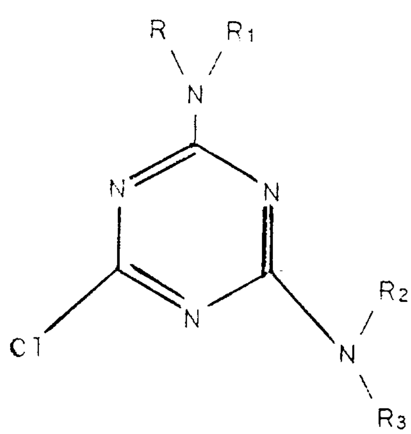

(V)

wherein the radicals R to $R_3$ have the meanings given in claim 1.

**3.** Process for preparing the compounds of general formula (I) according to claim 1, wherein an intermediate of general formula (VII):

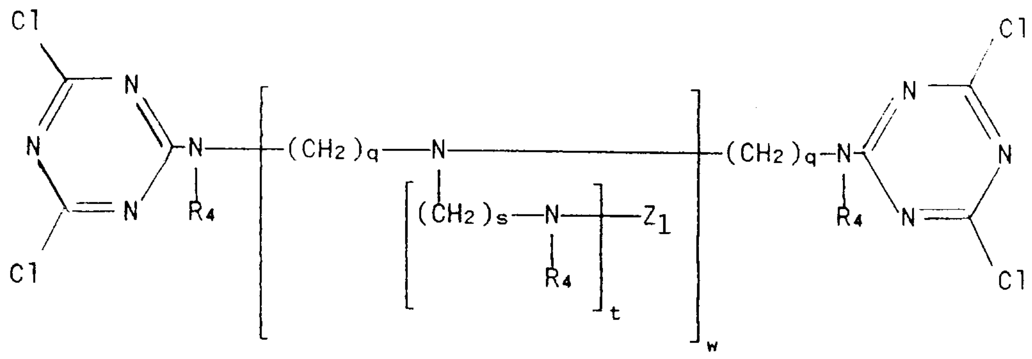

(VII)

wherein $R_4$, q, s, t and w have the meanings given in claim 1 and $Z_1$ is hydrogen or a group of formula

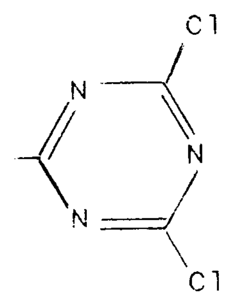

and its specific meaning can be different in each repeating unit; is reacted

(a) with at the most (2 + w) moles per mole of intermediate of an amine of general formula (II):

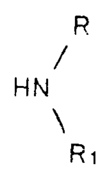

(II)

and then with at the most (2 + w) moles per mole of intermediate of an amine of general formula (IV):

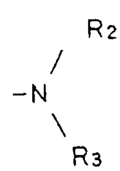

(IV)

wherein the radicals R to $R_3$ have the meanings given in claim 1;

or

(b) with at the most 2(2 + w) moles per mole of intermediate of an amine of general formula (II).

**Claims for the following Contracting State : ES**

**1.** Process for preparing triazinic compounds of general formula (I):

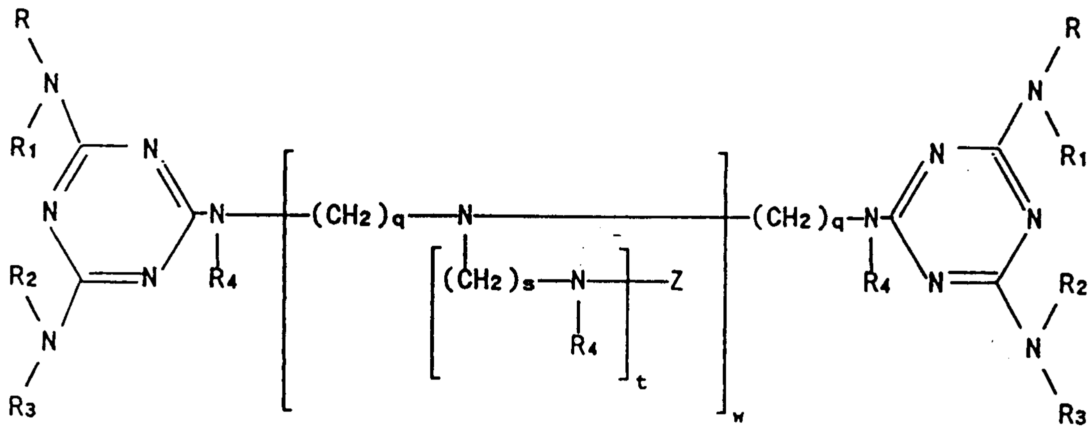

(I)

wherein:
t is 0 or 1;
when t is 0:
at least one of the radicals R, $R_1$, $R_2$ and $R_3$ is selected from

$-[C_nH_{2n}]-O-R_5$; and

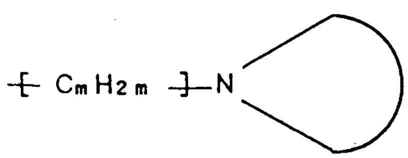

wherein:

- n = an integer of from 2 to 8;
- m = an integer of from 2 to 6;
- $R_5$ = H; $(C_1-C_8)$-alkyl; $(C_2-C_6)$-alkenyl; $-[C_pH_{2p}]-O-R_6$, wherein p is an integer of from 1 to 4 and $R_6$ is H or a $(C_1-C_4)$-alkyl group; $(C_6-C_{12})$-cycloalkyl; or $(C_6-C_{12})$-alkylcycloalkyl;

and the group:

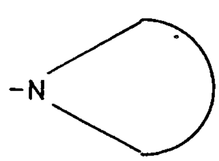

represents an N-heterocyclic radical which is selected from aziridinyl, pyrrolidinyl, piperidinyl, morpholinyl, thiomorpholinyl, piperazinyl, 4-methyl piperazinyl and 4-ethylpiperazinyl, and which is linked to the alkyl chain through the nitrogen atom;
or in general formula (I) at least one of the moieties

$NRR_1$ and $NR_2R_3$

represents an N-heterocyclic radical which is selected from aziridinyl, pyrrolidinyl, piperidinyl, morpholinyl, thiomorpholinyl, piperazinyl, 4-methyl piperazinyl, 4-ethyl piperazinyl, 2-methyl piperazinyl,

2,5-dimethyl piperazinyl, 2,3,5,6-tetramethyl piperazinyl, 2-ethyl piperazinyl and 2,5-diethyl piperazinyl, and which is linked to the triazine ring through the nitrogen atom;
the remaining radicals R to $R_3$, equal to or different from one another and having the same or different meanings in each triazine ring, are selected from the above groups and from H; $(C_1\text{-}C_{18})$-alkyl; $(C_2\text{-}C_8)$-alkenyl; $(C_6\text{-}C_{16})$-cycloalkyl or $(C_6\text{-}C_{16})$-alkylcycloalkyl, optionally substituted with one or more hydroxy and/or $(C_1\text{-}C_4)$-hydroxyalkyl groups;
<u>when t is 1</u>:
the radicals R to $R_3$, equal to or different from one another and having the same or different meanings in each triazine ring, are selected from H; $(C_1\text{-}C_{18})$-alkyl; $(C_2\text{-}C_8)$-alkenyl; $(C_6\text{-}C_{16})$-cycloalkyl or $(C_6\text{-}C_{16})$-alkylcycloalkyl, optionally substituted with one or more hydroxy and/or $(C_1\text{-}C_4)$-hydroxyalkyl groups;

$\left[ C_nH_{2n} \right] O\text{-}R_5$; and

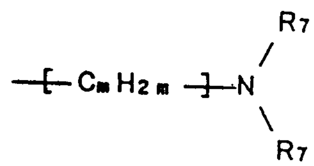

wherein:
n, m and $R_5$ are defined as above;
the radicals $R_7$, equal to or different from one another are selected from H; $(C_1\text{-}C_8)$-alkyl; $(C_2\text{-}C_6)$-alkenyl; $(C_6\text{-}C_{12})$-cycloalkyl; $(C_6\text{-}C_{12})$-alkylcycloalkyl; and $(C_1\text{-}C_4)$-hydroxyalkyl; and the moiety $N(R_7)_2$ may represent an N-heterocyclic radical which is selected from aziridinyl, pyrrolidinyl, piperidinyl, morpholinyl, thiomorpholinyl, piperazinyl, 4-methyl piperazinyl and 4-ethyl piperazinyl, and which is linked to the alkyl chain through the nitrogen atom;
or in general formula (I) at least one of the moieties

$NRR_1$ and $NR_2R_3$

represents an N-heterocyclic radical which is selected from aziridinyl, pyrrolidinyl, piperidinyl, morpholinyl, thiomorpholinyl, piperazinyl, 4-methyl piperazinyl, 4-ethyl piperazinyl, 2-methyl piperazinyl, 2,5-dimethyl piperazinyl, 2,3,5,6-tetramethyl piperazinyl, 2-ethyl piperazinyl and 2,5-diethyl piperazinyl, and which is linked to the triazine ring through the nitrogen atom;
$R_4$ is hydrogen or $(C_1\text{-}C_4)$-alkyl;
the subscripts <u>q</u>, equal to or different from each other, are integers of from 2 to 5;
s is an integer of from 2 to 4;
w is an integer of from 1 to 5; and
Z represents hydrogen or a group of general formula:

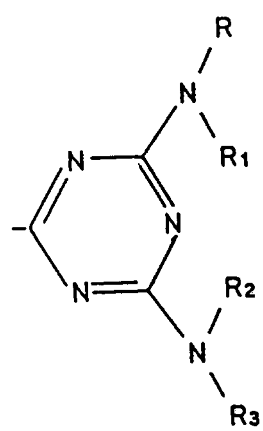

wherein R to $R_3$ are as defined above for t = 0 or t = 1, respectively, and the specific meanings of Z

can be different in each repeating unit,
wherein a polyamine of general formula (VI):

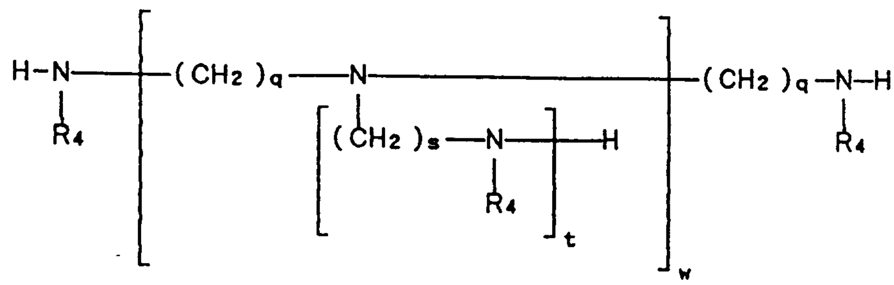

(VI)

wherein $R_4$, q, s, t and w have the meanings mentioned above, is reacted with at the most (2+w) moles per mole of polyamine of an intermediate of general formula (V):

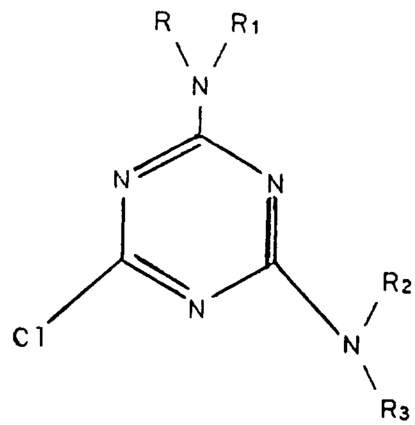

(V)

wherein the radicals R to $R_3$ have the meanings mentioned above.

**2.** Process for preparing triazinic compounds of general formula (I) as defined in claim 1, wherein an intermediate of general formula (VII):

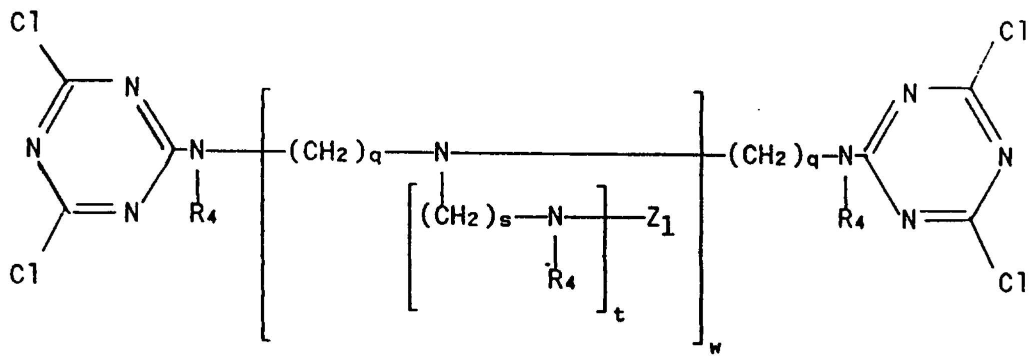

(VII)

wherein $R_4$, q, s, t and w have the meanings mentioned in claim 1 and $Z_1$ is hydrogen or a group of formula

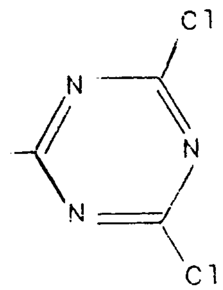

and its specific meaning can be different in each repeating unit; is reacted

(a) with at the most (2 + w) moles per mole of intermediate of an amine of general formula (II):

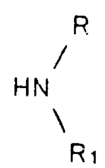

(II)

and then with at the most (2 + w) moles per mole of intermediate of an amine of general formula (IV):

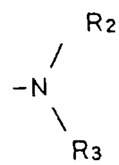

(IV)

wherein the radicals R to $R_3$ have the meanings mentioned in claim 1 or

(b) with at the most 2(2 + w) moles per mole of intermediate of an amine of general formula (II).

## Patentansprüche

### Patentansprüche für folgende Vertragsstaaten : AT, BE, CH, DE, DK, FR, GB, LI, NL, SE

**1.** Triazinverbindung der allgemeinen Formel (I):

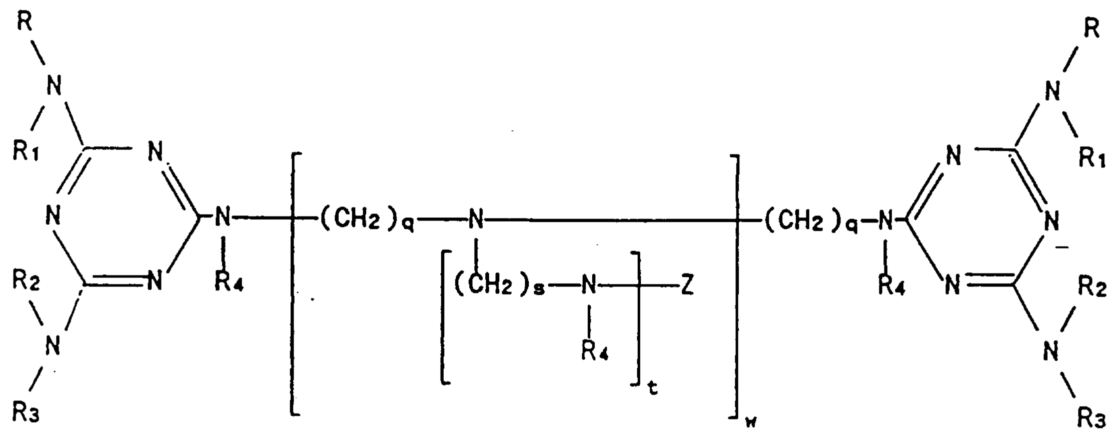

(I)

worin:
t = 0 oder 1 ist;
<u>wenn t = 0 ist:</u>
wenigstens eine der Gruppen R, $R_1$, $R_2$ und $R_3$ ausgewählt wird aus

$\{C_nH_{2n}\}O\text{-}R_5$; und

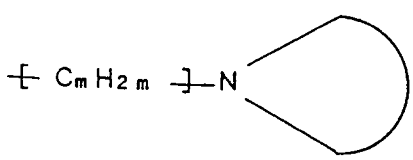

worin:

n = eine ganze Zahl von 2 bis 8 ist;
m = eine ganze Zahl von 2 bis 6 ist;
$R_5$ = H, ein ($C_1$-$C_8$-Alkyl, ein ($C_2$-$C_6$)-Alkenyl, $\{C_pH_{2p}\}O\text{-}R_6$, worin <u>p</u> eine ganze Zahl von 1 bis 4 und $R_6$ = H oder eine ($C_1$-$C_4$)-Alkylgruppe ist, ein ($C_6$-$C_{12}$)-Cycloalkyl oder ein ($C_6$-$C_{12}$)-Alkylcycloalkyl ist;

und die Gruppe:

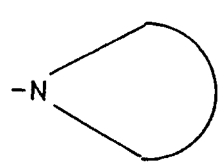

eine N-heterozyklische Gruppe, ausgewählt aus Aziridinyl, Pyrrolidinyl, Piperidinyl, Morpholinyl, Thiomorpholinyl, Piperazinyl, 4-Methylpiperazinyl und 4-Ethylpiperazinyl bedeutet, welche über das Stickstoffatom an die Alkylkette gebunden ist;
oder in der allgemeinen Formel (I) wenigstens eine der Gruppen

$NRR_1$ und $NR_2R_3$

eine N-heterozyklische Gruppe bedeutet, die ausgewählt wird aus: Aziridinyl, Pyrrolidinyl, Piperidinyl, Morpholinyl, Thiomorpholinyl, Piperazinyl, 4-Methylpiperazinyl, 4-Ethylpiperazinyl, 2-Methylpiperazinyl, 2,5-Dimethylpiperazinyl, 2,3,5,6-Tetramethylpiperazinyl, 2-Ethylpioperazinyl und 2,5-Diethylpiperazinyl,die über das Stickstoffatom an den Triazinring gebunden ist;
die verbleibenden Gruppen R bis $R_3$, die gleich oder verschieden sein und in jedem Triazinring die gleiche oder verschiedene Bedeutungen haben können, werden aus den obigen Gruppen und aus H, $(C_1-C_{18})$-Alkyl, $(C_2-C_8)$-Alkenyl, $(C_6-C_{16})$-Cycloalkyl oder $(C_6-C_{16})$-Alkylcycloalkyl, gegebenenfalls mit einem oder mehreren Hydroxy- und/oder $(C_1-C_4)$-Hydroxyalkyl-gruppen substituiert, ausgewählt;
wenn t = 1 ist:
die Gruppen R bis $R_3$, die gleich oder verschieden sein und die gleiche oder verschiedene Bedeutungen in jedem Triazinring haben können, werden ausgewählt aus:
H, $(C_1-C_{18})$-Alkyl, $(C_2-C_8)$-Alkenyl, $(C_6C_{16})$-Cycloalkyl oder $(C_6-C_{16})$-Alkylcycloalkyl, gegebenenfalls substituiert mit einer oder mehreren Hydroxy- und/oder $(C_1-C_4)$-Hydroxyalkylgruppen,

$-[C_nH_{2n}]-O-R_5$; und

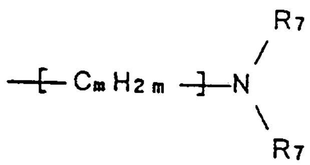

worin:
n, m und $R_5$ die oben definierten Bedeutungen haben; die Gruppen $R_7$, die gleich oder verschieden sein können, ausgewählt werden aus:
H, $(C_1-C_8)$-Alkyl, $(C_2-C_6)$-Alkenyl, $(C_6-C_{12})$-Cycloalkyl, $(C_6-C_{12})$-Alkylcycloalkyl und $(C_1-C_4)$-Hydroxyalkyl;
und die Gruppe $N(R_7)_2$ eine N-heterozyklische Gruppe bedeuten kann, die ausgewählt wird aus: Aziridinyl, Pyrrolidinyl, Piperidinyl, Morpholinyl, Thiomorpholinyl, Piperazinyl, 4-Methylpiperazinyl und 4-Ethylpiperazinyl, die über das Stickstoffatom an die Alkylkette gebunden ist;
oder in der allgemeinen Formel (I) wenigstens eine der Gruppen

$NRR_1$ und $NR_2R_3$

eine N-heterozyklische Gruppe bedeutet, die ausgewählt wird aus: Aziridinyl, Pyrrolidinyl, Piperidinyl, Morpholinyl, Thiomorpholinyl, Piperazinyl, 4-Methylpiperazinyl, 4-Ethylpiperazinyl, 2-Methylpiperazinyl, 2,5-Dimethylpiperazinyl, 2,3,5,6-Tetramethylpiperazinyl, 2-Ethylpiperazinyl und 2,5-Diethylpiperazinyl, die über das Stickstoffatom an den Triazinring gebunden ist;
$R_4$ = Wasserstoff oder $(C_1-C_4)$-Alkyl bedeutet;
die Indices q, die gleich oder verschieden sein können, ganze Zahlen von 2 bis 5 sind;
s = eine ganze Zahl von 2 bis 4 bedeutet;
w = eine ganze Zahl von 1 bis 5 bedeutet und
Z = Wasserstoff oder eine Gruppe der allgemeinen Formel:

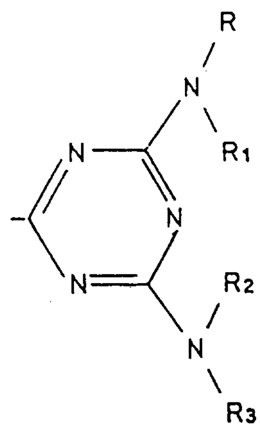

darstellt, worin R bis $R_3$ die oben definierten Bedeutungen haben, wenn t = 0 bzw. t = 1 ist, und wobei die jeweiligen Bedeutungen von Z in jeder wiederkehrenden Einheit verschieden sein können.

**2.** Verfahren zur Herstellung der Verbindungen der allgemeinen Formel (I) nach Anspruch 1, worin ein Polyamin der allgemeinen Formel (VI)

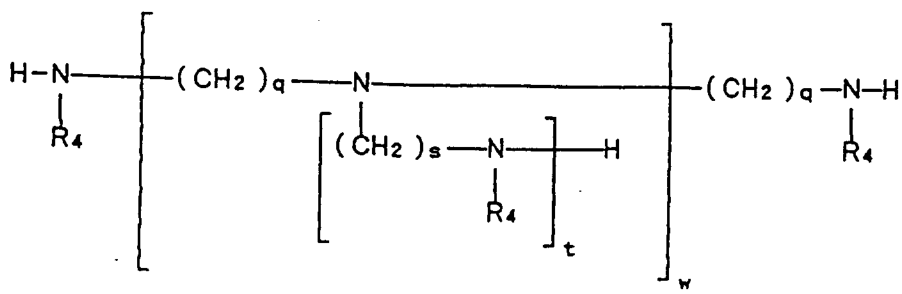

(VI)

worin $R_4$, q, s, t und w die in Anspruch 1 angegebenen Bedeutungen haben,
umgesetzt wird mit höchstens (2+w) Mol pro Mol Polyamin eines Zwischenprodukts der allgemeinen Formel (V):

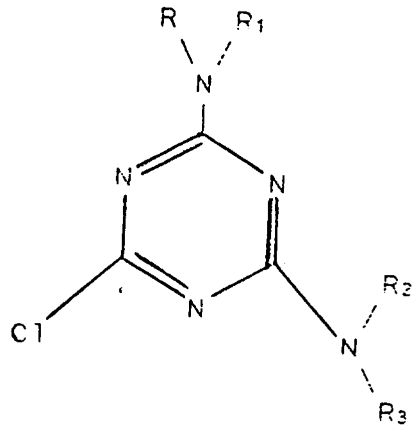

(V)

worin die Gruppen R bis $R_3$ die in Anspruch 1 definierten Bedeutungen haben.

3. Verfahren zur Herstellung der Verbindungen der allgemeinen Formel (I) nach Anspruch 1, worin ein Zwischenprodukt der allgemeinen Formel (VII):

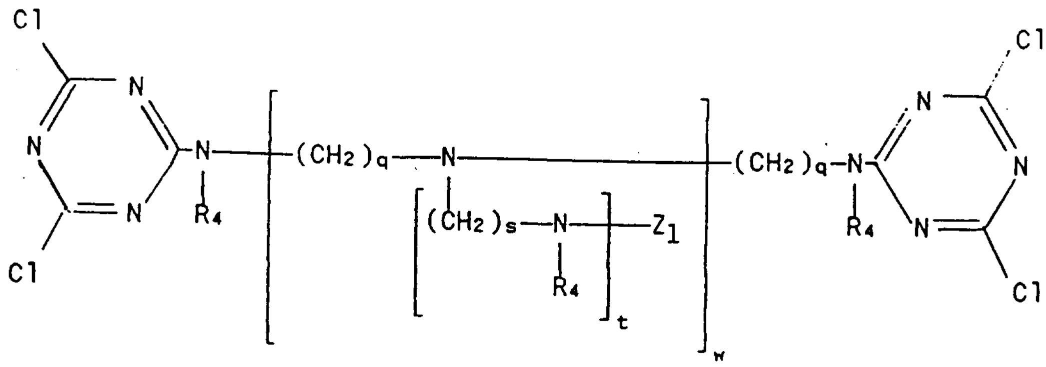

(VII)

worin $R_4$, q, s, t und w die in Anspruch 1 definierten Bedeutungen haben und $Z_1$ Wasserstoff oder eine Gruppe der Formel

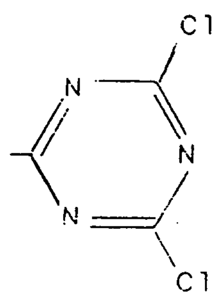

darstellt, wobei die jeweilige Bedeutung in jeder wiederkehrenden Einheit verschieden sein kann, umgesetzt wird

(a) mit höchstens (2 + w) Mol pro Mol des Zwischenprodukts eines Amins der allgemeinen Formel (II):

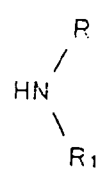

(II)

und dann mit höchstens (2 + w) Mol pro Mol eines Zwischenprodukts eines Amins der allgemeinen Formel (IV):

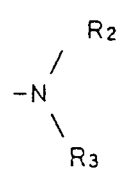

(IV)

worin die Gruppen R bis $R_3$ die in Anspruch 1 angegebenen Bedeutungen haben;
oder
(b) mit höchstens 2(2 + w) Mol pro Mol eines Zwischenprodukts eines Amins der allgemeinen Formel (II).

**Patentansprüche für folgenden Vertragsstaat : ES**

**1.** Verfahren zur Herstellung von Triazinverbindungen der allgemeinen Formel (I):

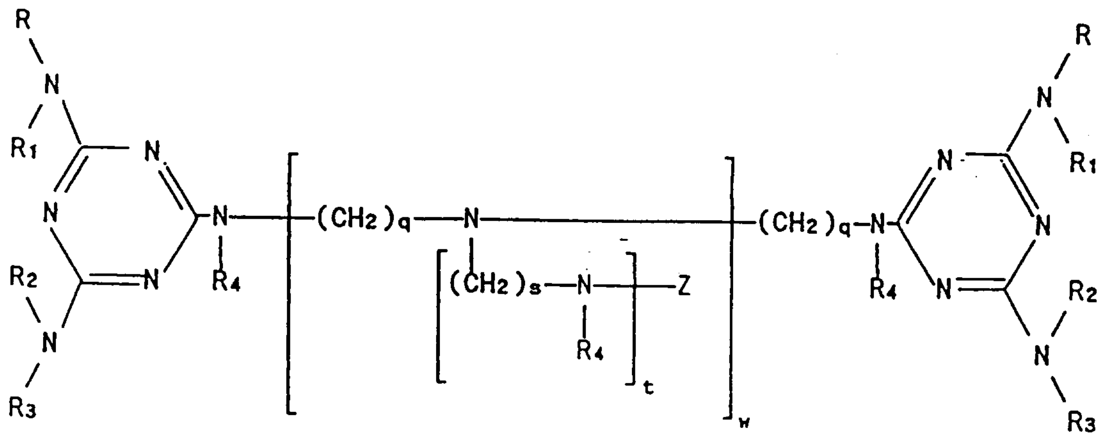

(I)

worin:
t = 0 oder 1 ist;
wenn t = 0 ist:
wenigstens eine der Gruppen R, $R_1$, $R_2$ und $R_3$ ausgewählt wird aus

$-[C_nH_{2n}]-O-R_5$ und

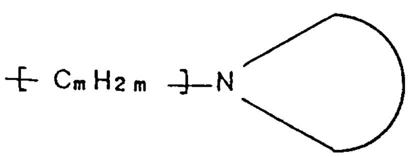

worin:

n = eine ganze Zahl von 2 bis 8 ist;
m = eine ganze Zahl von 2 bis 6 ist;
$R_5$ = H, ein ($C_1$-$C_8$)-Alkyl, ein ($C_2$-$C_6$)-Alkenyl, $-[C_pH_{2p}]-O-R_6$, worin p eine ganze Zahl von 1 bis 4 und $R_6$ = H oder eine ($C_1$-$C_4$)-Alkylgruppe ist, ein ($C_6$-$C_{12}$)-Cycloalkyl oder ein ($C_6$-$C_{12}$)-Alkylcycloalkyl ist;

und die Gruppe:

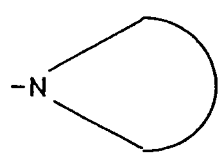

eine N-heterozyklische Gruppe, ausgewählt aus Aziridinyl, Pyrrolidinyl, Piperidinyl, Morpholinyl, Thiomorpholinyl, Piperazinyl, 4-Methylpiperazinyl und 4-Ethylpiperazinyl bedeutet, welche über das Stickstoffatom an die Alkylkette gebunden ist;
oder in der allgemeinen Formel (I) wenigstens eine der Gruppen

$NRR_1$ und $NR_2R_3$

eine N-heterozyklische Gruppe bedeutet, die ausgewählt wird aus: Aziridinyl, Pyrrolidinyl, Piperidinyl, Morpholinyl, Thiomorpholinyl, Piperazinyl, 4-Methylpiperazinyl, 4-Ethylpiperazinyl, 2-Methylpiperazinyl, 2,5-Dimethylpiperazinyl, 2,3,5,6-Tetramethylpiperazinyl, 2-Ethylpioperazinyl und 2,5-Diethylpiperazinyl,die über das Stickstoffatom an den Triazinring gebunden ist;
die verbleibenden Gruppen R bis $R_3$, die gleich oder verschieden sein und in jedem Triazinring die gleiche oder verschiedene Bedeutungen haben können, werden aus den obigen Gruppen und aus H, $(C_1$-$C_{18})$-Alkyl, $(C_2$-$C_8)$-Alkenyl, $(C_6$-$C_{16})$ - Cycloalkyl oder $(C_6$-$C_{16})$-Alkylcycloalkyl, gegebenenfalls mit einem oder mehreren Hydroxy- und/oder $(C_1$-$C_4)$--Hydroxyalkyl-gruppen substituiert, ausgewählt;
wenn t = 1 ist:
die Gruppen R bis $R_3$, die gleich oder verschieden sein und die gleiche oder verschiedene Bedeutungen in jedem Triazinring haben können, werden ausgewählt aus:
H, $(C_1$-$C_{18})$-Alkyl, $(C_2$-$C_8)$-Alkenyl, $(C_6C_{16})$-Cycloalkyl oder $(C_6$-$C_{16})$-Alkylcycloalkyl, gegebenenfalls substituiert mit einer oder mehreren Hydroxy- und/oder $(C_1$-$C_4)$-Hydroxyalkyl-gruppen,

$\{C_nH_{2n}\}O$-$R_5$ und

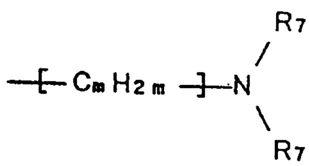

worin:
n, m und $R_5$ die oben definierten Bedeutungen haben;
die Gruppen $R_7$, die gleich oder verschieden sein können, ausgewählt werden aus:
H, $(C_1$-$C_8)$-Alkyl, $(C_2$-$C_6)$-Alkenyl, $(C_6C_{12})$-Cycloalkyl, $(C_6$-$C_{12})$-Alkylcycloalkyl und $(C_1$-$C_4)$-Hydroxyalkyl;
und die Gruppe $N(R_7)_2$ eine heterozyklische Gruppe bedeuten kann, die ausgewählt wird aus: Aziridinyl, Pyrrolidinyl, Piperidinyl, Morpholinyl, Thiomorpholinyl, Piperazinyl, 4-Methylpiperazinyl und 4-Ethylpiperazinyl, die über das Stickstoffatom an die Alkylkette gebunden ist;
oder in der allgemeinen Formel (I) wenigstens eine der Gruppen

$NRR_1$ und $NR_2R_3$

eine N-heterozyklische Gruppe bedeutet, die ausgewählt wird aus: Aziridinyl, Pyrrolidinyl, Piperidinyl, Morpholinyl, Thiomorpholinyl, Piperazinyl, 4-Methylpiperazinyl, 4-Ethylpiperazinyl, 2-Methylpiperazinyl, 2,5-Dimethylpiperazinyl, 2,3,5,6-Tetramethylpiperazinyl, 2-Ethylpiperazinyl und 2,5-Diethylpiperazinyl, die über das Stickstoffatom an den Triazinring gebunden ist;
$R_4$ = Wasserstoff oder $(C_1$-$C_4)$-Alkyl bedeutet;
die Indices q, die gleich oder verschieden sein können, ganze Zahlen von 2 bis 5 sind;
s = eine ganze Zahl von 2 bis 4 bedeutet;
w = eine ganze Zahl von 1 bis 5 bedeutet und
Z = Wasserstoff oder eine Gruppe der allgemeinen Formel:

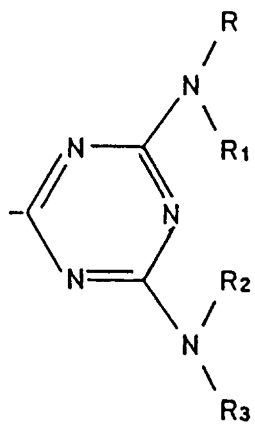

darstellt, worin R bis $R_3$ die oben definierten Bedeutungen haben, wenn t = 0 bzw. t = 1 ist, und wobei die jeweiligen Bedeutungen von Z in jeder wiederkehrenden Einheit verschieden sein können, worin ein Polyamin der allgemeinen Formel (VI):

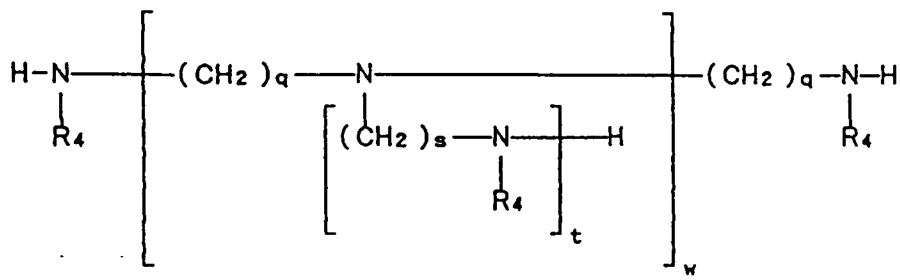

(VI)

worin $R_4$, q, s, t und w die oben definierten Bedeutungen haben, umgesetzt wird mit höchstens (2+w) Mol pro Mol Polyamin eines Zwischenprodukts der allgemeinen Formel (V):

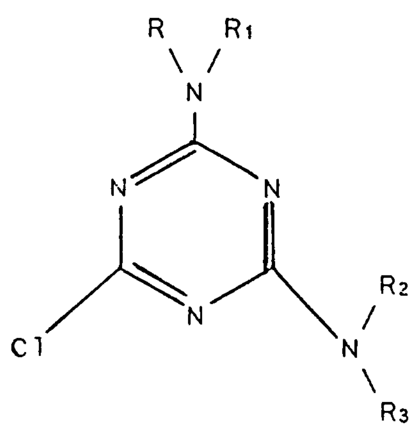

(V)

worin die Gruppen R bis $R_3$ die oben definierten Bedeutungen haben.

2. Verfahren zur Herstellung von Triazinverbindungen der allgemeinen Formel (I), wie sie in Anspruch 1 definiert wurde, worin ein Zwischenprodukt der allgemeinen Formel (VII):

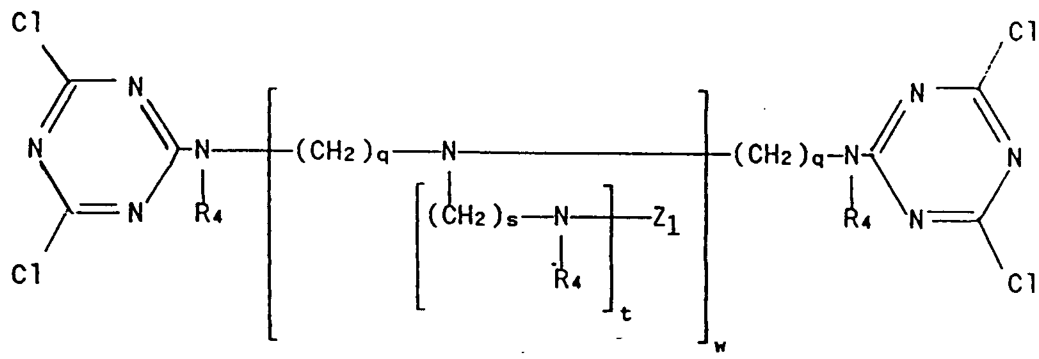

(VII)

worin $R_4$, q, s, t und w die in Anspruch 1 angegebenen Bedeutungen haben und $Z_1$ = Wasserstoff oder eine Gruppe der Formel

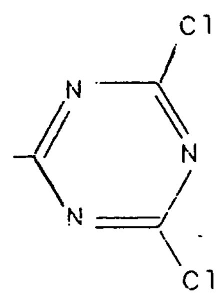

bedeutet, wobei die jeweilige Bedeutung in jeder wiederkehrenden Einheit verschieden sein kann, umgesetzt wird

(a) mit höchstens (2 + w) Mol pro Mol des Zwischenprodukts eines Amins der allgemeinen Formel (II):

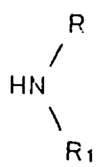

(II)

und dann mit höchstens (2 + w) Mol pro Mol eines Zwischenprodukts eines Amins der allgemeinen Formel (IV):

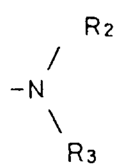

(IV)

worin die Gruppen R bis $R_3$ die in Anspruch 1 angegebenen Bedeutungen haben;
oder

(b) mit höchstens 2(2 + w) Mol pro Mol eines Zwischenprodukts eines Amins der allgemeinen Formel (II).

**Revendications**

**Revendications pour les Etats contractants suivants : AT, BE, CH, DE, DK, FR, GB, LI, NL, SE**

**1.** Composés triaziniques de formule générale (I):

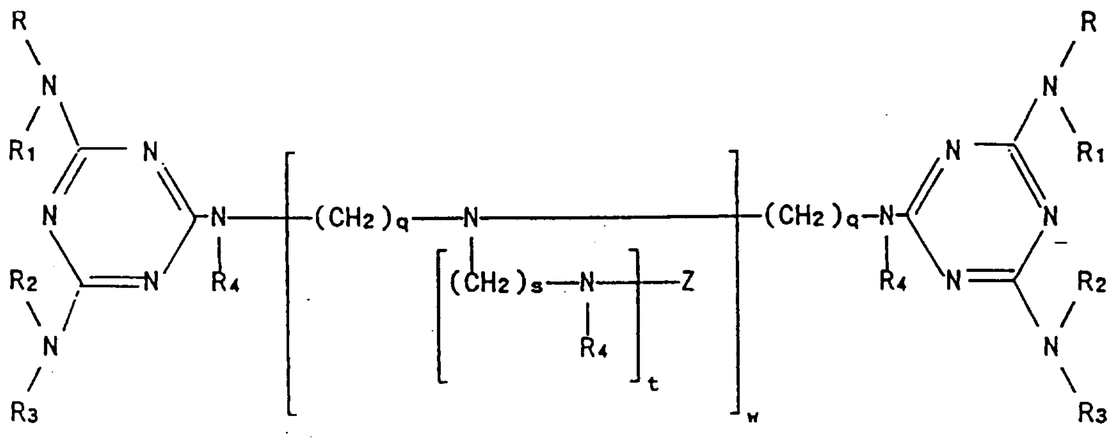

(I)

dans laquelle:
t est égal à 0 ou 1;
lorsque t est égal à 0: au moins l'un des radicaux R, $R_1$, $R_2$ et $R_3$ consiste en

$-[-C_nH_{2n}-]-O-R_5$ ou en

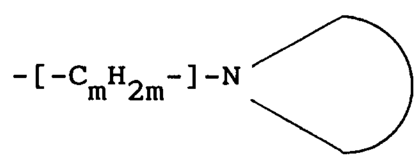

dans lesquelles:

- n est un nombre entier compris entre 2 et 8, de préférence entre 2 et 4, bornes comprises;
- m est un nombre entier compris entre 2 et 6, bornes comprises;
- $R_5$ est un atome d'hydrogène; un groupe alkyle en $C_1$ à $C_8$; alkényle en $C_2$ à $C_6$; $-[-C_pH_{2p}-]-O-R_6$ dans lequel:
- p est un nombre entier compris entre 1 et 4, bornes comprises; et
- $R_6$ est un atome d'hydrogène ou un radical alkyle
- en $C_1$ à $C_4$; cycloalkyle en $C_6$ à $C_{12}$; ou alkylcycloalkyle en $C_6$ à $C_{12}$; et le groupe:

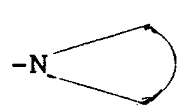

représente un radical N-hétérocyclique qui est lié à la chaîne alkyle par l'atome d'azote, et qui est choisi dans le groupe comprenant aziridinyle, pyrrolidinyle, piperidinyle, morpholinyle, thiomorpholinyle, piperazinyle, 4-méthylpiperazinyle et 4-éthylpiperazinyle;
ou dans la formule générale (I), au moins l'une des entités:

$NRR_1$ et $NR_2R_3$

représente un radical N-hétérocyclique qui est lié au cycle triazine par un atome d'azote, et qui est choisi parmi les radicaux aziridinyle, pyrrolidinyle, piperidinyle, morpholinyle, thiomorpholinyle, piperazinyle, 4-méthylpiperazinyle, 4-éthylpiperazinyle, 2-méthylpiperazinyle, 2,5-diméthylpiperazinyle, 2,3,5,6-tétraméthylpiperazinyle, 2-éthylpipérazinyle, 2-éthylpiperazinyle et 2,5-diéthylpiperazinyle;
les autres radicaux R à $R_3$, identiques ou différents les uns des autres, présentant des significations identiques ou différentes dans chaque cycle triazine, sont choisis dans les groupes précités et également parmi: un atome d'hydrogène; un radical alkyle en $C_1$ à $C_{18}$; un radical alkényle en $C_2$ à$C_8$; un radical cycloalkyle en $C_6$ à $C_{16}$ ou alkylcycloalkyle en $C_6$ à $C_{16}$, éventuellement substitué par un ou plusieurs groupes hydroxy et/ou hydroxyalkyle en $C_1$ à $C_4$;
lorsque t est égal à 1:
les radicaux R à $R_3$, identiques ou différents les uns des autres et présentant la même ou différentes significations dans chaque cycle triazine, sont choisi dans le groupe comprenant un atome d'hydrogène; un radical alkyle en $C_1$ à $C_{18}$; un radical alkényle en $C_2$ à $C_8$; un radical cycloalkyle en $C_6$ à $C_{16}$ ou alkylcycloalkyle en $C_6$ à $C_{16}$, éventuellement substitué par un ou plusieurs groupes hydroxy et/ou hydroxyalkyle en $C_1$ à $C_4$;

$-[C_nH_{2n}]-O-R_5$ et

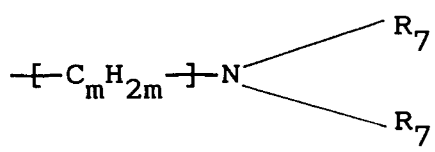

dans lesquels n, m et $R_5$ sont tels que définis ci-dessus;
les radicaux $R_7$, identiques ou différents les uns des autres, sont choisis dans le groupe comprenant un atome d'hydrogène; un groupe alkyle en $C_1$ à $C_8$; alkényle en $C_2$ à $C_6$; cycloalkyle en $C_6$ à $C_{12}$; alkylcycloalkyle en $C_6$ à $C_{12}$; hydroxyalkyle en $C_1$ à $C_4$; et
l'entité $N(R_7)_2$ peut représenter un radical N-hétérocyclique qui est lié à la chaîne alkyle par l'atome d'azote et qui est choisi dans le groupe comprenant les radicaux aziridinyle, pyrrolidinyle, piperidinyle, morpholinyle, thiomorpholinyle, pipérazinyle, 4-péthylpipérazinyle et 4-éthylpiperazinyle;
ou dans la formule générale (I), au moins l'une des entités $NRR_1$ et $NR_2R_3$
représente un radical N-hétérocyclique qui est lié au cycle triazine par l'atome d'azote et qui est choisi dans le groupe comprenant les radicaux aziridinyle, pyrrolidinyle, pipéridinyle, morpholinyle, thiomorpholinyle, pipérazinyle, 4-méthyl-piperazinyle, 4-éthyl-piperazinyle, 2-méthylpiperazinyle, 2,5-diméthylpiperazinyle, 2,3,5,6-tétraméthylpiperazinyle, 2-éthylpiperazinyle et 2,5-diéthylpiperazinyle;

$R_4$ est un atome d'hydrogène ou un radical alkyle en $C_1$ à $C_4$,

les indices q, identiques ou différents les uns des autres,
sont des nombres entiers compris entre 2 et 5, bornes incluses;

s est un nombre entier compris entre 2 et 4, bornes incluses;

w est un nombre entier compris entre 1 et 5, bornes incluses; et

Z est un atome d'hydrogène ou un groupe de formule générale:

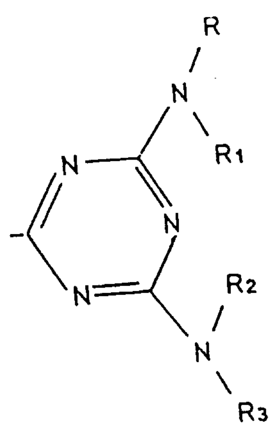

dans laquelle:

R à $R_3$ sont tels que définis ci-dessus et ceci en relation avec les définitions de t = 0 ou t = 1, respectivement, les significations spécifiques de Z pouvant différer dans chaque unité répétitive.

2. Procédé de préparation des composés de formule générale (I) selon la revendication 1, dans lequel une polyamine de formule générale (VI):

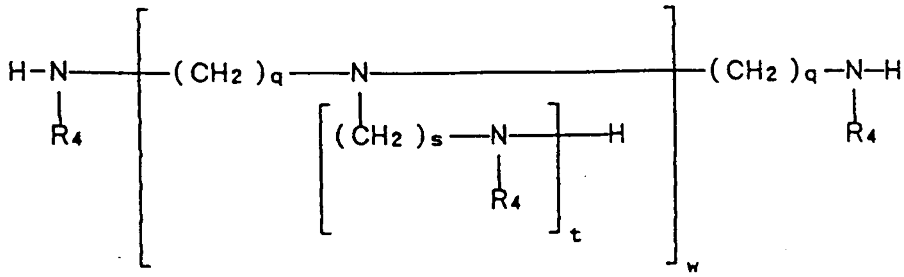

(VI)

dans laquelle $R_4$, q, s, t et w ont les significations données dans la revendication 1, réagit avec au plus (2 + w) moles par mole de polyamine d'intermédiaire de formule générale (V):

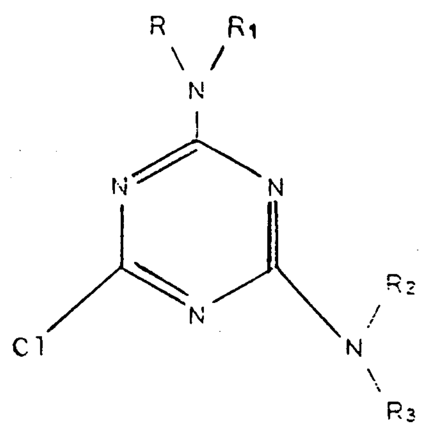

(V)

dans laquelle les radicaux R à $R_3$ ont les mêmes significations que celles données dans la revendication 1.

3. Procédé de préparation de composés de formule générale (I) selon la revendication 1, dans lequel un intermédiaire de formule générale (VII):

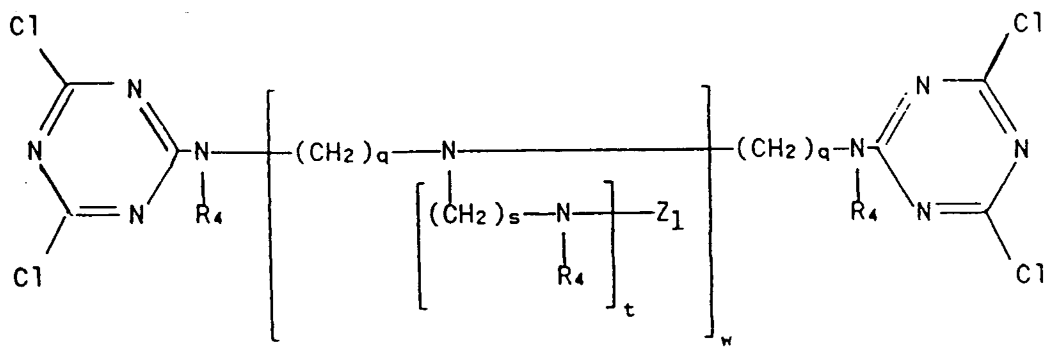

(VII)

dans laquelle:

$R_4$, q, s, t et w ont les significations indiquées dans la revendication 1; et

$Z_1$ est un atome d'hydrogène ou un groupe de formule:

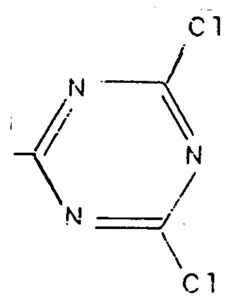

et dont la signification peut être différente dans chaque unité répétitive, réagit avec:

(a) au plus (2 + w) moles par mole d'intermédiaire d'une amine de formule générale (II):

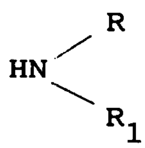

(II)

et avec au plus (2 + w) moles par mole d'intermédiaire d'une amine de formule générale (IV):

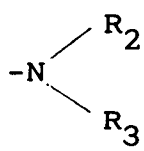

(IV)

dans laquelle les radicaux R à $R_3$ ont les significations indiquées dans la revendication 1;

ou

(b) avec au plus 2(2 + w) moles par mole d'intermédiaire d'une amine de formule générale (II).

**Revendications pour l'Etat contractant suivant : ES**

**1.** Procédé de préparation des composés triaziniques de formule générale (I):

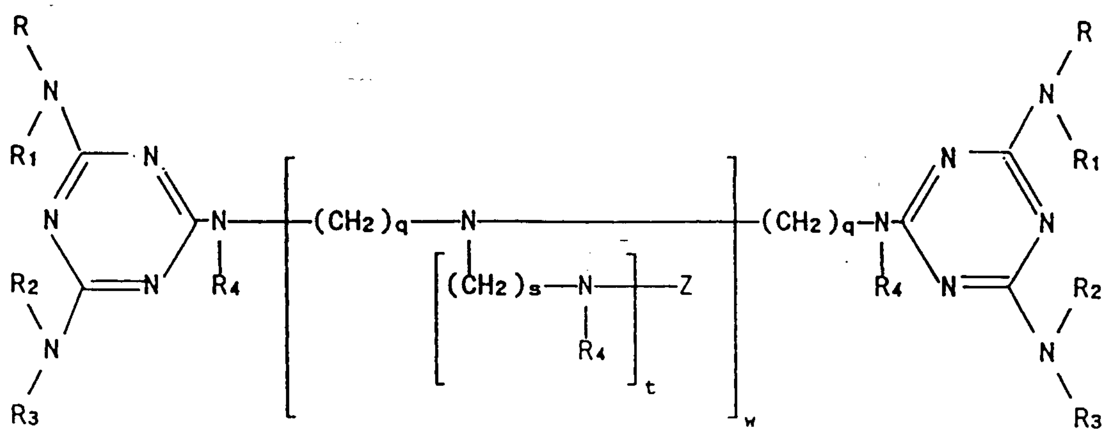

(I)

dans laquelle:
t est ègal à 0 ou 1;
lorsque t est égal à 0: au moins l'un des radicaux R, $R_1$, $R_2$ et $R_3$ consiste en

$-[-C_nH_{2n}-]-O-R_5$ ou en

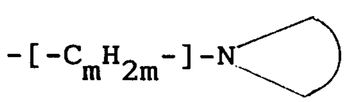

dans lesquelles:

- n est un nombre entier compris entre 2 et 8, de préférence entre 2 et 4, bornes comprises;
- m est un nombre entier compris entre 2 et 6, bornes comprises;
- $R_5$ est un atome d'hydrogène; un groupe alkyle en $C_1$ à $C_8$; alkényle en $C_2$ à $C_6$; $-[-C_pH_{2p}-]-O-R_6$ dans lequel:
- p est un nombre entier compris entre 1 et 4, bornes comprises; et
- $R_6$ est un atome d'hydrogène ou un radical alkyle en $C_1$ à $C_4$; cycloalkyle en $C_6$ à $C_{12}$; ou alkylcycloalkyle en $C_6$ à $C_{12}$; et le groupe:

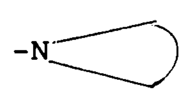

représente un radical N-hétérocyclique qui est lié à la chaîne alkyle par l'atome d'azote, et qui est choisi dans le groupe comprenant aziridinyle, pyrrolidinyle, piperidinyle, morpholinyle, thiomorpholinyle, piperazinyle, 4-méthylpiperazinyle et 4-éthylpiperazinyle;
ou dans la formule générale (I), au moins l'une des entités:

$NRR_1$ et $NR_2R_3$

représente un radical N-hétérocyclique qui est lié au cycle triazine par un atome d'azote, et qui est choisi parmi les radicaux aziridinyle, pyrrolidinyle, piperidinyle, morpholinyle, thiomorpholinyle, piperazinyle, 4-méthylpiperazinyle, 4-éthylpiperazinyle, 2-méthylpiperazinyle, 2,5-diméthyipiperazinyle, 2,3,5,6-tétraméthylpiperazinyle, 2-éthylpipérazinyle, 2-éthylpiperazinyle et 2,5-diéthylpiperazinyle;
les autres radicaux R à $R_3$, identiques ou différents les uns des autres, présentant des significations

identiques ou différentes dans chaque cycle triazine, sont choisis dans les groupes précités et également parmi: un atome d'hydrogène; un radical alkyle en $C_1$ à $C_{18}$; un radical alkényle en $C_2$ à $C_8$; un radical cycloalkyle en $C_6$ à $C_{16}$ ou alkylcycloalkyle en $C_6$ à $C_{16}$, éventuellement substitué par un ou plusieurs groupes hydroxy et/ou hydroxyalkyle en $C_1$ à $C_4$; lorsque t est égal à 1:
les radicaux R à $R_3$, identiques ou différents les uns des autres et présentant la même ou différentes significations dans chaque cycle triazine, sont choisi dans le groupe comprenant un atome d'hydrogène; un radical alkyle en $C_1$ à $C_{18}$; un radical alkényle en $C_2$ à $C_8$; un radical cycloalkyle en $C_6$ à $C_{16}$ ou alkylcycloalkyle en $C_6$ à $C_{16}$, éventuellement substitué par un ou plusieurs groupes hydroxy et/ou hydroxyalkyle en $C_1$ à $C_4$;

$-[-C_nH_{2n}-]-O-R_5$ et

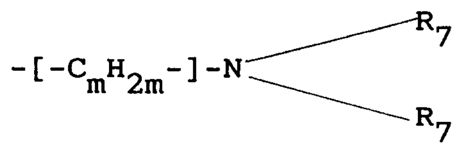

dans lesquels n, m et $R_5$ sont tels que définis ci-dessus;

les radicaux $R_7$, identiques ou différents les uns des autres, sont choisis dans le groupe comprenant un atome d'hydrogène; un groupe alkyle en $C_1$ à $C_8$; alkényle en $C_2$ à $C_6$; cycloalkyle en $C_6$ à $C_{12}$; alkylcycloalkyle en $C_6$ à $C_{12}$; hydroxyalkyle en $C_1$ à $C_4$; et

l'entité $N(R_7)_2$ peut représenter un radical N-hétérocyclique qui est lié à la chaîne alkyle par l'atome d'azote et qui est choisi dans le groupe comprenant les radicaux aziridinyle, pyrrolidinyle, piperidinyle, morpholinyle, thiomorpholinyle, pipérazinyle, 4-péthylpipérazinyle et 4-éthylpiperazinyle;

ou dans la formule générale (I), au moins l'une des entités $NRR_1$ et $NR_2R_3$
représente un radical N-hétérocyclique qui est lié au cycle triazine par l'atome d'azote et qui est choisi dans le groupe comprenant les radicaux aziridinyle, pyrrolidinyle, pipéridinyle, morpholinyle, thiomorpholinyle, pipérazinyle, 4-méthylpiperazinyle, 4-éthyl-piperazinyle, 2-méthylpiperazinyle, 2,5-diméthylpiperazinyle, 2,3,5,6-tétraméthylpiperazinyle, 2-éthylpiperazinyle et 2,5-diéthylpiperazinyle;

$R_4$ est un atome d'hydrogène ou un radical alkyle en $C_1$ à $C_4$,
les indices q, identiques ou différents les uns des autres,
sont des nombres entiers compris entre 2 et 5, bornes incluses;

s est un nombre entier compris entre 2 et 4, bornes incluses;
w est un nombre entier compris entre 1 et 5, bornes incluses; et
Z est un atome d'hydrogène ou un groupe de formule générale:

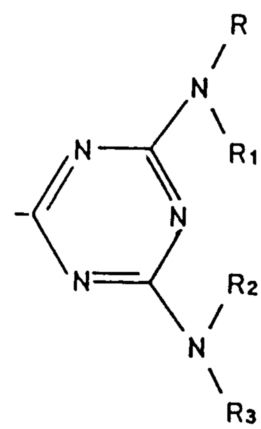

dans laquelle:

R à $R_3$ sont tels que définis ci-dessus et ceci en relation avec les définitions de t = 0 ou t = 1, respectivement, les significations spécifiques de Z pouvant différer dans chaque unité répétitive

dans lequel une polyamine de formule générale (VI):

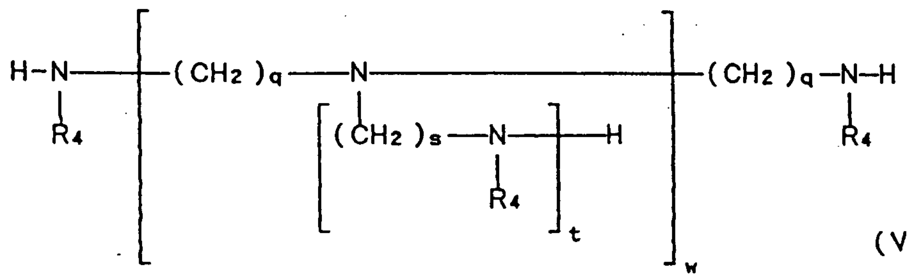

(VI)

dans laquelle $R_4$, q, s, t et w ont les significations données ci-dessus, réagit avec au plus (2 + w) moles par mole de polyamine d'un intermédiaire de formule générale (V):

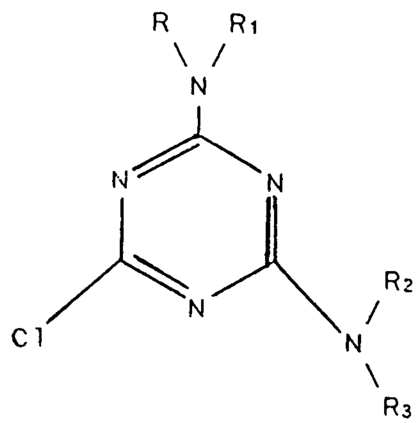

(V)

dans laquelle les radicaux R à $R_3$ ont les mêmes significations que celles indiquées ci-dessus.

**2.** Procédé de préparation des composés triaziniques de formule générale (I) tels que définis dans la revendication 1, dans lequel un intermédiaire de formule générale (VII):

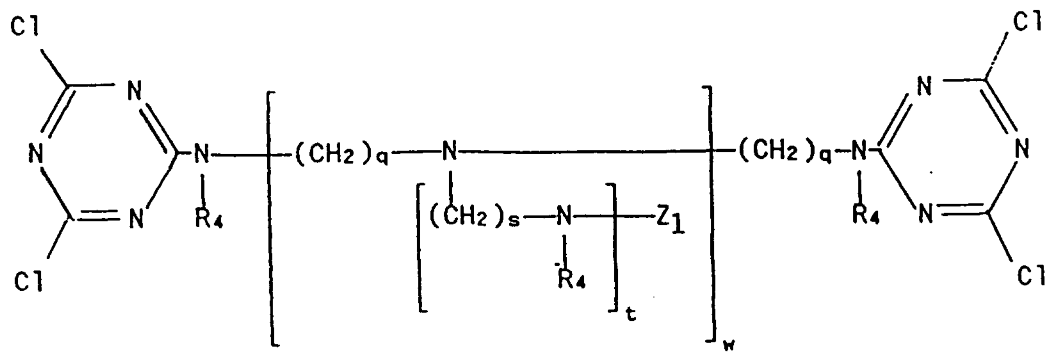

(VII)

dans laquelle:

$R_4$, q, s, t et w ont les significations indiquées dans la revendication 1; et

$Z_1$ est un atome d'hydrogène ou un groupe de formule:

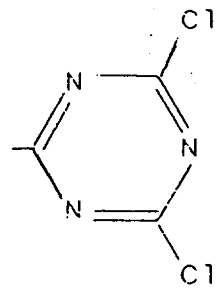

et dont la signification peut être différente dans chaque unité répétitive, réagit avec:

(a) au plus (2 + w) moles par mole d'un intermédiaire d'une amine de formule générale (II):

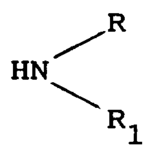

(II)

et avec au plus (2 + w) moles par mole d'intermédiaire d'une amine de formule générale (IV):

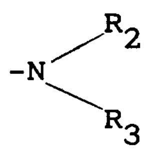

(IV)

dans laquelle les radicaux R à $R_3$ ont les significations indiquées dans la revendication 1;

ou

(b) avec au plus 2(2 + w) moles par mole d'intermédiaire d'une amine de formule générale (II).